(19) Europäisches Patentamt
European Patent Office
Office européen des brevets

(11) **EP 2 640 439 B1**

(12) **EUROPÄISCHE PATENTSCHRIFT**

(45) Veröffentlichungstag und Bekanntmachung des Hinweises auf die Patenterteilung:
**05.06.2019 Patentblatt 2019/23**

(51) Int Cl.:
*A61M 1/16* (2006.01)      *A61M 1/34* (2006.01)

(21) Anmeldenummer: **11819054.5**

(22) Anmeldetag: **15.11.2011**

(86) Internationale Anmeldenummer:
**PCT/DE2011/001989**

(87) Internationale Veröffentlichungsnummer:
**WO 2012/095066 (19.07.2012 Gazette 2012/29)**

(54) **VORRICHTUNG ZUR BESTMÖGLICHEN ERREICHUNG DES SUBSTITUTIONSZIELS BEI DER ULTRAFILTRATION VON BLUT**

DEVICE FOR ACHIEVING, AS CLOSELY AS POSSIBLE, THE SUBSTITUTION TARGET IN BLOOD ULTRAFILTRATION

DISPOSITIF POUR ATTEINDRE LE MIEUX POSSIBLE L'OBJECTIF DE SUBSTITUTION LORS DE L'ULTRAFILTRATION DU SANG

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.11.2010 DE 102010052070**

(43) Veröffentlichungstag der Anmeldung:
**25.09.2013 Patentblatt 2013/39**

(73) Patentinhaber: **B. Braun Avitum AG**
**34212 Melsungen (DE)**

(72) Erfinder:
• **WOLFF, Henrik**
 **37213 Witzenhausen (DE)**

• **MOLL, Stefan**
 **22587 Hamburg (DE)**

(74) Vertreter: **Winter, Brandl, Fürniss, Hübner, Röss, Kaiser, Polte - Partnerschaft mbB Patent- und Rechtsanwaltskanzlei Alois-Steinecker-Strasse 22 85354 Freising (DE)**

(56) Entgegenhaltungen:
EP-A1- 1 175 917      EP-A2- 0 240 101
WO-A1-2007/140993     US-A1- 2005 203 493
US-A1- 2008 215 247   US-B1- 7 131 956

**EP 2 640 439 B1**

**Beschreibung**

Die vorliegende Erfindung betrifft eine Blutbehandlungseinheit zur Ausführung eines Verfahrens zur bestmöglichen Erreichung eines vorher berechneten Substitutionsziels bei der Ultrafiltration von Blut. Erfindungsgemäß wird eine Blutbehandlungseinheit zur Ausführung eines Verfahrens bereitgestellt, wobei durch die Messung von Druckmesswerten in der Blutbehandlungseinheit festgestellt wird, ob das berechnete Substitutionsziel am Ende der Dialysesitzung erreicht wird oder nicht und bei dem Resultat, dass das berechnete Substitutionsziel mit den momentanen Dialyseparametern nicht erreicht werden kann, erfolgt eine Anpassung der Flussrate von Blut, Dialysat und/oder Substituat, um bei festgelegter Behandlungszeit das Substitutionsziel bestmöglichst zu erreichen oder bei festgelegtem Substitutionsziel die Behandlungsdauer möglichst wenig zu verändern.

Die Erfindung bezieht sich dabei auf das Feld der Filtration, genauer gesagt der Tangentialflussfiltration TFF. Hierbei ist die zu reinigende Flüssigkeit durch eine semipermeable Membran von der Reinigungslösung getrennt. Die Reinigungslösung wird auch als Dialyselösung oder Dialyseflüssigkeit bezeichnet. Die Dialyseflüssigkeit hat je nach Bedarf verschiedene Eigenschaften durch die gelösten Stoffe, die zur Dialyse genutzt werden. Die Konzentration der Verbindungen in der Dialyseflüssigkeit hängt von der Art der Verbindungen in der Flüssigkeit, der Dialysezeit, dem Alter und Körpergewicht des Patienten, den zu bekämpfenden Symptomen und anderen Bedingungen ab. Dialyselösungen werden im Allgemeinen vor Gebrauch aus Dialysekonzentrat und Wasser hergestellt. Typischerweise weist die Reinigungslösung eine niedrigere Konzentration derjenigen Stoffe auf, die aus der zu reinigenden Flüssigkeit entfernt werden sollen, als die zu reinigende Flüssigkeit selbst. Durch diesen Konzentrationsgradienten wird über eine Austauschoberfläche Diffusion erzeugt. Um die Diffusion als reinigende Kraft optimal nutzen zu können, werden Tangentialflussfilter typischerweise im Gegenstromprinzip betrieben.

Ein Dialysefilter besteht im Wesentlichen aus Hohlfasern, also zylinderförmigen Fasern, die länglich gestreckt ein Gehäuse durchziehen. Die Wände der Hohlfasern wirken dabei durch teildurchlässige (semipermeable) Strukturen als Membranen. An ihren Enden sind die Hohlfasern in eine Vergussmasse eingebettet. Im Dialysefilter können die Hohlfasern zu Modulen mit mehreren Quadratmetern Filterfläche zusammengefasst werden. Bei der Dialyse durch Tangentialflussfiltration, auch Querstromfiltration oder Cross-Flow-Filtration genannt, wird den Hohlfasern über einen ersten Flüssigkeitskreislauf Blut/Plasma zugeführt, das sie der Länge nach durchströmt. Durch einen zweiten Flüssigkeitskreislauf wird die Dialyseflüssigkeit, in der Regel im Gegenstromprinzip, aber ggf. auch parallel zum Blutstrom zugeführt. Das Gehäuse weist also vier Anschlüsse auf, und zwar für jeden Flüssigkeitsstrom zwei, jeweils einen zur Zufuhr und zur Abfuhr. An der Innenseite der Hohlfasermembran befindet sich also der Blutstrom, und auf der Außenseite die Dialyseflüssigkeit.

Ein weiterer Reinigungsmechanismus ist die Konvektion. Hier wird ein Druckgradient über die semipermeable Membran erzeugt, wodurch die zu reinigende Flüssigkeit über die semipermeable Membran gedrückt wird. Dadurch werden die Substanzen in ihrer vorliegenden Konzentration mit gespült. Dieser Reinigungsprozess ist also nicht von der Konzentration an Substanzen in der Reinigungslösung abhängig, entscheidend sind hier nur die Konzentration in der zu reinigenden Flüssigkeit und die Membraneigenschaften wie Siebkoeffizient, Permeabilität usw.. Es ist also von Interesse die Filtereigenschaften zu Behandlungsbeginn sowie während der Behandlung zu kennen.

Ein spezieller Bereich der Filtration ist die extrakorporale Blutbehandlung bei chronischer oder akuter Niereninsuffizienz. Hier ist die zu reinigende Flüssigkeit das Blut des Patienten und die Reinigungslösung ist die Dialyseflüssigkeit. Entscheidend in diesem speziellen Fall der TFF ist es in den Behandlungen (im chronischen Fall typischerweise drei pro Woche) die Blutreinigungsfunktion der Niere möglichst effektiv zu ersetzen. Um dies zu gewährleisten, hat sich der Kt/V-Wert als Maß für die Behandlungsqualität durchgesetzt. Der Kt/V ist ein Parameter, um die Dialyseeffektivität zu bestimmen und ein wesentlicher Bestandteil zur Beurteilung der Dialyseeffizienz. K ist die Clearance, die über den Harnstoffgehalt des Blutes vor und nach der Dialyse ermittelt wird. Der Wert t zeigt die effektive Dialysezeit in Minuten und der Wert V das Harnstoffverteilungsvolumen. Dies ist der Körperwassergehalt, der ca. 60% der Körpermasse darstellt. Ziel einer Behandlung ist es einen Kt/V $\geq$ 1,2 zu erzielen. In einem normalen Behandlungsverlauf werden Werte erzielt, die dieses Kriterium in der Regel erfüllen. Allerdings können in einer Behandlung Widrigkeiten auftreten, die sich negativ auf den Behandlungsverlauf sowie das Behandlungsergebnis auswirken. Es ist somit wichtig während einer Behandlung die Einflussparameter zu überwachen und zu kontrollieren, um auf solche Widrigkeiten schnell und vor allem gezielt reagieren zu können und die Systemparameter während der Dialyse entsprechend anzupassen.

Bei modernen Dialysatoren wird eine Reinigung durch Ausnutzen der Konvektion durch das Prinzip der Ultrafiltration erreicht. Hierbei werden nicht nur die zu entfernenden oder harnpflichtigen Substanzen aus dem Blut entfernt. Aufgrund des über die Ultrafiltrationspumpe (UF-Pumpe) angelegten Druckgradienten (Transmembrandruck) an der Filtermembran wird dem Blut konvektiv Plasma über die Membran entzogen. Zu diesem Zweck wird auf der Dialysatseite aus einem geschlossenen System eine definierte Menge Dialyselösung entnommen. Dadurch wird in dem geschlossenen System ein Unterdruck erzeugt, der dafür sorgt, das die gleiche Menge Blut über die semipermeable Membran zur Dialysatseite übertritt.

Der Volumenverlust an Plasma muß durch Zuführung von Substituatlösung ausgeglichen werden. Die Substituatlösung ist typischerweise eine Elektrolytlösung. Die Ultrafiltrationsrate (UF-Rate) beschreibt das Volumen des derart entzogenen Blutplasmas pro Zeiteinheit und somit auch das Volumen an Substituatlösung, das dem Blut wiederzugeführt werden muß. Die Zumischung des Substituats erfolgt entweder vor dem Dialysator (Prädilution) oder nach dem Dialysator (Postdilution). Als Obergrenze für die Substitutionslösung gelten typischerweise 25-30% des Blutflusses bei der Postdilution bei einer Hämodiafiltration (HDF). Im Predilution Modus besteht diese Beschränkung nicht.

Die Substitution gleicht also die über den Flüssigkeitsentzug durch die Ultrafiltration entnommene Flüssigkeit kontinuierlich aus und vermeidet dadurch Volumenverluste. Die Substitutionsrate wird dabei durch ein bestimmtes Volumen pro Zeiteinheit definiert.

Die gebrauchte Dialyselösung wird jeweils durch ein gleiches Volumen an frischer Dialyselösung ersetzt. Dies kann in einer sog. Bilanzkammer erfolgen. Moderne System erreichen hierbei eine maximale Abweichung von 0,07% während einer mehrstündigen Dialysesitzung. Um ein Vermischen der verbrauchten mit der frischen Dialyselösung zu verhindern, sind die beiden Kammern durch eine Gummimembran voneinander getrennt.

Ein entscheidender Prozess ist die Interaktion der Filtermembran mit Blut. Durch diese Wechselwirkung verschlechtern sich die Flusseigenschaften des Filters sowohl in Transmembranrichtung als auch in Blutflussrichtung. Diese Veränderungen werden beispielsweise durch Thrombozytenanlagerung auf die Membran, Koagelbildung, chemisches Binden von Blutbestandteilen an die Membran oder schlicht mechanisches (strömungsbedingtes) drücken der Blutbestandteile an und sogar in die Membran verursacht.

Transmembranrichtung oder transmembranös bezieht sich hierbei auf einen Fluss des Blutes über die Membran des Dialysators bzw. Dialysefilters.

Bei der Koagelbildung entsteht ein gallertartiges durch Fibrinfäden stabilisiertes Aggregat aus roten Blutkörperchen (Erythrozyten). Anders als der Begriff Thrombus beschreibt ein Koagulum ein Blutgerinnsel, welches sich außerhalb eines Blut- oder Lymphgefäßes (extravasal) befindet und nicht innerhalb (intravasal).

Diese und weitere Änderungen der Systemeigenschaften haben diverse Auswirkungen auf den Behandlungsverlauf und die Behandlungsqualität. Besonders die Behandlung durch Hämodiafiltration wird dadurch beeinträchtigt, da hier auf den konvektiven Stofftransport mittelmolekularer Substanzen gesetzt wird. Die besonders durch die Ablagerungen an der Filtermembran veränderten Fluß- und Strömungseigenschaften führen zu einem veränderten Bedarf an Dialyselösung. Durch Verschlechterung der transmembranösen Flusseigenschaften oder der Permeabilität, verschlechtert sich auch der Siebkoeffizient für die urämischen Substanzen im mittelmolekularen Bereich, was dazu führt, dass bei gleicher Menge konvektiv gefilterter Flüssigkeit weniger urämische Substanzen aus dem Blutkreislauf entfernt werden. Ein anderer Effekt ist die Verringerung der effektiven Durchflussfläche sowohl in Blutflussrichtung als auch in Transmembranrichtung. Dies hat eine Reduzierung der aktiven Filteroberfläche zur Folge, wodurch es zu einer Verschlechterung der diffusiven Reinigung kommen kann. Bei neuwertigen Filtern besteht in der Regel ein Pufferpotential, das größer ist als die maximale physiologische Filterung. Dadurch kann eine Verringerung der effektiven Durchflussfläche in einem bestimmten Maß abgefangen werden. Ist dieses Potential jedoch ausgeschöpft, kommt es zu dem zuvor beschriebenen Effekt.

Als geeignete Gegenmaßnahmen oder Reaktionen auf solche Änderungen gelten allgemein das Spülen mit Kochsalzlösung zum "Säubern" des Dialysators, die Zugabe von Heparin, um weitere Koagelbildung zu verhindern oder das Senken der Ultrafiltrationsrate (UF), um die Hämokonzentration zu reduzieren. Die Permeabilität der Membranen wird bestimmt durch Messung des Flüssigkeitsvolumens, das bei gegebener Druckdifferenz bei einer Temperatur von 37°C durch eine festgelegte Membranfläche durch die Membran tritt und das zur allgemeinen Vergleichbarkeit auf Flächeneinheit, Zeiteinheit und Druckeinheit normiert wird. Als Flüssigkeit zur Bestimmung der Ultrafiltrationsrate wird Wasser verwendet.

Aus dem Stand der Technik sind bereits Versuche bekannt, die das Ziel haben Systemänderungen zu erkennen und darauf zu reagieren. In der US 2008/0215247 A1 wird davon ausgegangen, dass der lineare Zusammenhang zwischen Transmembrandruck (TMP) und Ultrafiltrationsrate OuF = TMP • KuF (KuF = Ultrafiltrationskoeffizient) nur in einem gewissen Bereich des TMPs erfüllt ist. Somit wird zunächst die Funktion OuF(TMP) abgeschätzt, indem der TMP sukzessive erhöht und die dadurch erzeugte Ultrafiltrationsrate gemessen wird. Ab einem bestimmten Wert hat eine Erhöhung des TMP eine immer geringere Steigerung der Ultrafiltrationsrate zur Folge. Man wählt daher den Kniepunkt (Tangentialpunkt) der Funktion OuF(TMP) als Arbeitspunkt Da sich der KuF während der Behandlung auf Grund der Systemänderung verschlechtert, offenbart die WO 2006/011009 A2 die technischen Voraussetzungen, den Zusammenhang zwischen TMP und OuF stündlich neu ermitteln zu lassen.

In der EP 1175917 A1 wird als weiteres Konzept das Anpassen der Verhältnisse aus Prä- und Postdilution im Verlaufe der Behandlung beschrieben. Die beidem Begriffe Prä- und Postdilution beziehen sich darauf, an welcher Stelle - vor oder nach dem Durchlauf des Blutes durch den Dialysefilter - eine Volumensubstitution des Blutes vorzugsweise mit einer Elektrolytlösung und damit eine Dilution des Blutes durchgeführt wird.

Weiterer Stand der Technik findet sich in der US 7 131 956 B1, welche eine Dialysemaschine beschreibt, deren Berechnungseinheit abhängig von der Ultrafiltrationsraten und einer Plasmaflussrate einen Filtrationsfaktor berechnet, diesen mit einem Grenzwert vergleicht und das Resultat mittels einer Signaleinrichtung anzeigt.

Da die Substituatlösung direkt in das Blut des Patienten geleitet wird, liegt es in der Natur der Sache, für die Substituatlösung sterile, pyrogenfreie und unter GMP (good manufacturing practice) - Bedingungen hergestellte Komponenten bzw. Konzentrate von infusionsgerechter Qualität zu nutzen. Dabei werden Verfahren wie In-Line-Durchdämpfung aller Teile der Herstellungsanlagen, Keimfiltration des Produktes, Inprocess-Kontrollen (Filterdichte, Konzentrationskontrolle), quantitative Endkontrolle und Dokumentation von Herstellung und Qualitätskontrolle. Die Zusammensetzung der Substituatlösung entspricht dabei in etwa jener der Dialyseflüssigkeit. Die aufwändige Herstellung von Substituatlösungen verursachen hohe Produktionskosten, die sich in den finanziellen Aufwendungen für Dialysebehandlungen niederschlagen.

Unterschiedliche Dialysatoren bzw. Filter bedingen auf Grund verschiedener Eigenschaften unterschiedliche Dialyseverläufe und verbrauchen unterschiedliche Mengen an Dialyse- und Substituatflüssigkeit. Die Information über die verwendeten Dialysatoren und besonders über die in den verwendeten Dialysatoren installierten Filter, sind dem bedienenden Personal oft nicht bekannt, so dass der Dialyseverlauf und besonders die benötigten Volumina an Dialyseflüssigkeit, besonders aber an Substituatflüssigkeit, nicht genau bestimmt werden können.

Der Bedarf an Dialyse- und Substituatflüssigkeit variiert weiterhin in Abhängigkeit vom Dialyseverlauf, so dass ein gewünschtes Substitutionsziel oftmals nicht erreicht werden kann. Es besteht darum Bedarf an einer Vorrichtung und einem Verfahren, welches zum einen die Filtereigenschaften bestimmen und in die Berechnung eines auf den jeweiligen Patienten abgestimmten Substitutionsziels einfließen lassen kann, weil das Substitutionsziel wesentlich durch die Filtereigenschaften bestimmt wird. Zum anderen soll die Vorrichtung und das Verfahren in der Lage sein, das berechnete Substitutionsziel durch Anpassung von Dialyseparametern am Ende der Dialysesitzung bestmöglichst zu erreichen.

Wie hierin verwendet bezeichnet der Begriff "Dialysator", welcher synonym zu "Transmembranfilter" ist, das Gehäuse mit Einlaß und Auslaß für die Dialyseflüssigkeit sowie Einlaß und Auslaß für das Blut, wobei sich in dem Gehäuse der Filter befindet. Als Filter werden vorzugsweise Hohlfasern verwendet, welche im Inneren durch die Dialyseflüssigkeit durchflossen werden und außen entlang der Hohlfasern das Blut fließt und die zu entfernenden Stoffe durch Diffusion und Konvektion aus dem Blut durch die porösen Hohlfasern in die Dialyseflüssigkeit übertreten. Der Begriff "Filtereigenschaften" bezieht sich auf die Eigenschaften des Dialysators bzw. Transmembranfilters in Bezug auf die Blutreinigung. Als Filtereigenschaften sind vor allem die Oberfläche des Filters, sowie die Porendichte und die Porengröße sowie die Porengrößenverteilung entscheidend.

**Kurzbeschreibung der Erfindung**

Diese Aufgabe wird erfindungsgemäß durch die Blutbehandlungseinheit gemäß Anspruch 1 gelöst. Weitere vorteilhafte Ausgestaltungen der Erfindung ergeben sich aus den abhängigen Ansprüchen, der Beschreibung, den Figuren sowie den Beispielen.

Die vorliegende Erfindung bezieht sich auf ein Verfahren zur Berechnung oder Festlegung eines Substitutionsziels in Form des benötigten Volumens an Substitutionsflüssigkeit in Blutbehandlungseinheiten durch die Bestimmung von Druckmesswerten sowie auf eine Blutbehandlungseinheit, welche dieses Verfahren ausführen kann. Die Erfindung bezieht sich insbesondere auf eine Vorrichtung, nämlich eine Blutbehandlungseinheit, welche derart ausgelegt ist, ein vorher bestimmtes und festgelegtes Substitutionsziel durch Überprüfung des Dialyseverlaufs und nötigenfalls Anpassung der Dialyseparameter zu erreichen und dabei eine veranschlagte Dauer der Dialysesitzung möglichst wenig zu verändern, insbesondere möglichst wenig zu verlängern oder bei einer festgelegten Dauer der Dialysesitzung von z.B. 4 Stunden, 4,5 Stunden oder 5 Stunden, das Substitutionsziel bestmöglich zu erreichen. Erfindungsgemäß ist die Blutbehandlungseinheit nicht nur derart ausgelegt, ein berechnetes Substitutionsziel in der gegebenen Zeit bestmöglichst zu erreichen oder ein festgelegtes Substitutionsziel zu erreichen und die veranschlagte Behandlungsdauer möglichst wenig zu verändern, sondern auch ein für den jeweiligen Patienten bevorzugtes und an den gerade verwendeten Dialysator bzw. Filter angepasstes Substitutionsziel zu berechnen. Da die Dialysatoren Einwegartikel und damit Verbrauchsmaterialien sind, kommt es häufig vor, dass ein Patient bei verschiedenen Dialysesitzungen auch verschiedene Filter bzw. Dialysatoren erhält. Da das Substitutionsziel entscheidend vom jeweiligen Filter abhängt, ist die Blutbehandlungseinheit so ausgelegt, dass vor der eigentlichen Dialysesitzung die Eigenschaften des gerade verwendeten Filters durch eine Referenzlösung bestimmt werden. Sind die Kenndaten des Filters, was vor allen die effektive Filteroberfläche als auch die Permeabilität ist, auf Speichereinheit der Blutbehandlungseinheit hinterlegt, so kann mittels der Referenzlösung auch der genaue Filtertyp und nicht nur die Filtereigenschaften bestimmt werden. Anhand der so bestimmten Filtereigenschaften oder sogar des konkreten Filtertyps wird ein geeignetes Substitutionsziel berechnet. Geeignet bedeutet dabei nicht, ein maximales Substitutionsziel von beispielsweise 30L pro Dialysesitzung, d.h. pro Dialysebehandlung. Da ein geeignetes Substitutionsziel auch noch auf den jeweiligen Patienten abgestimmt sein sollte, werden vorzugsweise die Patientendaten aus früheren Dialysesitzungen wie beispielsweise in früheren Dialysesitzungen erreichte Substitutionsziele mit berücksichtigt oder auch Daten einer mit dem jeweiligen Patienten ähnliche oder vergleichbare Patientengruppe. So ergeben sich in der Regel geeignete Substitutionsziele im Bereich von 15L bis 28L, weiter bevorzugt 17L bis 27L, weiter bevorzugt 19L bis 26L, und noch weiter bevorzugt 21L bis 25L pro Dialysesitzung. Während der folgenden Dialysesitzung wird entweder das Substitutionsziel als Ziel, das zu erreichen ist festgesetzt und dabei von einer veranschlagten Behandlungsdauer möglichst wenig abgewichen oder die Behandlungsdauer wird festgesetzt und von den berechneten geeigneten Substitutionsziel ist möglichst wenig abzuweichen. Ob die Behandlungszeit bei festgelegtem Substitutionsziel eingehalten werden kann oder das Substitutionsziel bei festgelegter Behandlungszeit erreicht wird, wird durch Aufnahme von Druckmesswerten an mindestens zwei Drucksensoren während der Blutbehandlung ermittelt, welche mit Referenzdruckmesswerten verglichen werden, welche vorliegen sollten, wenn das Substitutionsziel am Ende der Blutbehandlung erreicht werden wird. Zeigt dieser Vergleich ein Übereinstimmen der gemessenen Druckmesswerte innerhalb eines Toleranzbereichs mit den Referenzdruckmesswerten, so wird voraussichtlich das Substitutionsziel an Ende der Blutbehandlung erreicht und die Dialyseparameter sind in Ordnung. Zeigt hingegen der Vergleich der gemessenen Druckmesswerte mit den Referenzdruckmesswerten innerhalb eines Toleranzbereichs eine Abweichung über den Toleranzbereich hinaus, so wird das Substitutionsziel am Ende der Dialysesitzung nicht erreicht und die Dialyseparameter müssen nachreguliert werden. Als für die Nachregulation geeignete Dialyseparameter werden die Flussrate des Blutes, die Flussrate des Dialysats (d.h. der Dialyseflüssigkeit) und die Flussrate des Substituats verwendet. Somit ist gewährleistet, dass für den jeweiligen Patienten eine optimale Dialysesitzung erfolgt, welche nicht zwingend mit einer optimalen Blutreinigung gleichzusetzen ist, sondern mit einer am besten verträglichen Dialysebehandlung für den jeweiligen Patienten bei der Verwendung eines bestimmten Filters bzw. Dialysators. Keine Blutbehandlungseinheit des Standes der Technik ermöglicht die Bestimmung der Filtereigenschaften oder des konkreten Filtertyps vor der Blutbehandlung zur Berechnung eines für den Patienten am besten verträglichen Substitutionsziels für die jeweilige Dialysesitzung unter Berücksichtigung der Filtereigenschaften und sieht Ausgestaltungen vor, welche die Dialyseparameter nachregulieren, um dieses am besten verträgliche Substitutionsziel am Ende der Blutbehandlung auch zu erreichen.

**Beschreibung der Erfindung**

Die vorliegende Erfindung betrifft eine Blutbehandlungseinheit mit einem Dialysator, mindestens zwei Drucksensoren, einer zentralen Recheneinheit und einer Speichereinheit, geeignet um folgendes Verfahren auszuführen:

a) Bestimmung der Filtereigenschaften durch Ermittlung von mindestens zwei Druckmesswerten an mindestens zwei Drucksensoren innerhalb der Blutbehandlungseinheit mit einer Referenzlösung,
b) optional Vergleich der gemessenen Druckmesswerte mit auf der Speichereinheit hinterlegten Referenzwerten desselben Patienten, welche in früheren Dialysesitzungen erhoben wurden oder mit auf der Speichereinheit hinterlegten Referenzwerten einer Patientengruppe,
c) Berechnung eines Substitutionsziels auf Grundlage der Werte gemäß Schritt a) und optional Schritt b),
d) Messen von Druckmesswerten an mindestens zwei Drucksensoren während einer Blutbehandlung,
e) Vergleich der gemäß Schritt d) gemessenen Druckmesswerte mit auf der Speichereinheit hinterlegten Referenzdruckmesswerten, und
f) Anpassung der Flussrate von Blut, Dialysat und/oder Substituat bei Abweichung von mindestens einem gemessenen Druckmesswert von dem Referenzdruckmesswert, um bei festgelegter Behandlungszeit das Substitutionsziel bestmöglichst zu erreichen oder bei festgelegtem Substitutionsziel die Behandlungsdauer möglichst wenig zu verändern.

Die Erfindung ist vorteilhaft, weil sie die Bestimmung der Filtereigenschaften zu Beginn der Dialyse erfasst und die Vorherbestimmung des wahrscheinlichen Dialyseverlaufs erlaubt. Dadurch können bereits vor Beginn der eigentlichen Behandlung der bestmögliche Verlauf der Dialyse und der Bedarf an Substitutionsflüssigkeit bestimmt werden.

Als Referenzdruckmesswerte dienen vorzugsweise solche, die während einer Blutbehandlung erhoben wurden, in der das Substitutionsziel erreicht worden ist.

Die Erfindung bezieht sich besonders auf eine Vorrichtung zur Ausführung eines Verfahrens, bei dem aus Druckmesssignalen an einer Blutbehandlungseinheit ein optimales Substitutionsziel zu Beginn einer Dialyse ermittelt wird und bei im Verlauf der Dialysesitzung auftretenden Störungen gegebenenfalls bestimmte Parameter nachgeregelt werden können. Das optimale Substitutionsziel wird durch das notwendige, vorbestimmte Volumen an Substituat definiert, das dem Blutkreislauf wieder zugeführt wird bzw. werden soll. Das substituierte Volumen kann dabei durch die Substitutionsrate über die Zeit des Flüssigkeitsaustauschs berechnet werden. Dabei ist erfindungswesentlich, dass die Substitutionsrate dynamisch an veränderte Bedingungen angepasst wird, da diese das Optimum während der Dialysesitzung verändern können.

Die Substitutionsrate wird dabei durch ein bestimmtes Volumen pro Zeiteinheit definiert. Die Substitutionsrate kann beispielsweise durch ml/min oder l/h ausgedrückt werden. Als zugeführtes Volumen des Substituats werden 1 - 10 l pro Stunde Behandlung zugeführt werden, bevorzugt 1 - 9, bevorzugter 2 - 8, noch bevorzugter 2 - 7,5, noch bevorzugter 2,5 - 7, noch bevorzugter 2,5 - 6,5, noch bevorzugter 2,5 - 6, noch bevorzugter 3 - 5,5 und am meisten bevorzugt 4 - 5 l/h. Dabei beträgt die Dialysesitzung insgesamt zwischen 4 und 5 Stunden, so dass das Substitutionsziel in der Regel bei 20L bis 26L pro Dialysesitzung liegt.

Das Substitutionsziel kann vorzugsweise aus der Blutflussrate, also dem strömenden Blutvolumen pro Zeiteinheit, bestimmt werden gemäß:

$$Qsub = [(1/3 \; {}^*BF \; {}^* T) - WL]^* \, p \qquad (1)$$

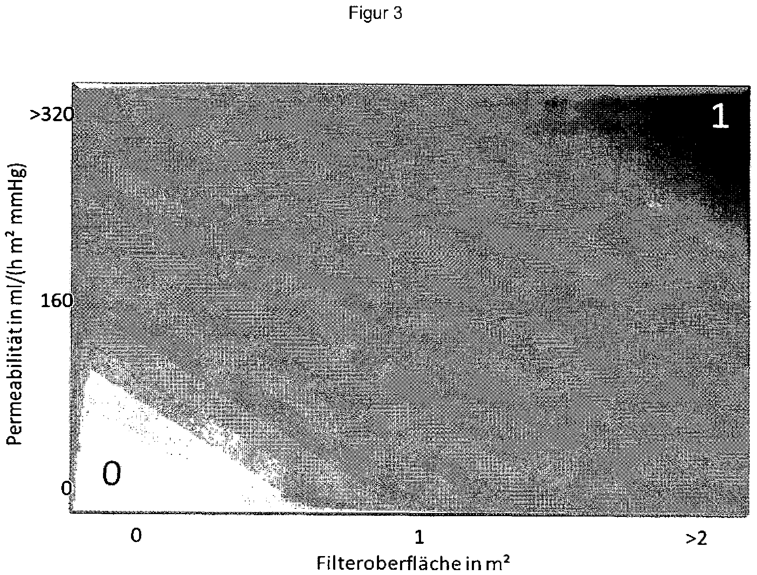

wobei BF die Blutflussrate, T die Dauer der Behandlung und p ein flussspezifischer Faktor ist, der durch die Druckmessungen mit einer Referenzlösung bestimmt wird und den mathematischen Zusammenhang zwischen Druckmesswerten und Flussrate wiedergibt. Der Faktor p kann aus einer Matrix ermittelt werden, in der Permeabilität und Filteroberfläche gegeneinander aufgetragen werden (Figur 3).

Das Substitutionsziel kann vorzugsweise auch über einen maximalen Hämatokrit bestimmt werden gemäß:

$$Qsub = [BF^*\{1 - [Hct(ein)/Hct(max)]\} - WL]^*p, \qquad (2)$$

wobei WL der Gewichtsverlust ist, der durch die Flüssigkeitsmenge entsteht, die dem Patienten während der Behandlung effektiv entzogen wurde und nicht substituiert wird. Der Hämatokrit wird über Sensoren erfasst, die an der Dialysevorrichtung vorhanden sein können oder vorhanden sind, wenn der Hämatokrit für die Berechnung des Substitutionsziels verwendet werden soll.

Das Substitutionsziel kann vorzugsweise auch über einen vorher bestimmten, nicht membrangängigen Blutbestandteil oder eine Gruppe von Bestandteilen X bestimmt werden, also große Proteine, Blutzellen u.ä. gemäß:

$$Qsub = [BF^*\{1 - [X(ein)/X(max)]\} - WL]^*p, \qquad (3)$$

Wichtig ist dabei zu beachten, dass nicht genau dieselbe Menge der dem Blut entzogenen Flüssigkeit an Substituat wieder dem Blut zugesetzt wird, sondern dass bei der Dialyseprozedur dem Patienten auch überschüssige Flüssigkeit entzogen wird, die er nicht ausscheiden kann, und die als sogenannte "weight loss" (Gewichtsverlust) bezeichnet wird. Das Substituat und der "weight loss" bilden das sogenannte konvektive Volumen.

Der Begriff Flussrate bezieht sich erfindungsgemäß auf die strömenden Flüssigkeiten, also Blut, Dialyseflüssigkeit sowie Substituatflüssigkeit. Die Flussrate wird durch das strömende Volumen pro Zeiteinheit definiert. Die Flussraten sind die wesentlichen Steuergrößen, um die Hämefiltration zu beeinflussen. Die Flussraten, also die Stärke der Strömung, kann dabei durch die blutseitigen bzw. dialysatseiligen Pumpen beeinflusst werden. Durch die Flussraten von Blut und Dialyseflüssigkeit (Dialysat) wird der TMP bestimmt, also der Druck, der an der Kapillarmembran den Flüssigkeitsdurchtritt bewirkt. Prinzipiell lässt sich der TMP aus der Ultrafiltrationsrate (Gewichtsabnahme) und dem Ultrafiltrationstaktor (Porengröße der Kapillarmembran) berechnen. Noch genauer und im Sinne der Erfindung bevorzugt lässt sich der TMP durch die Drücke auf der Blut- und Dialyseflüssigkeitsseite berechnen, nämlich durch

$$TMP = (PB1 + PB2)/2 - (PD1 + PD2)/2,$$

wobei PB1 der Druck auf der blutseitigen Eingangsseite ist, PB2 der Druck auf der blutseitigen Ausgangsseite, PD1 der Druck auf der Eingangsseite der Dialyseflüssigkeit und PD2 der Druck auf der Ausgangseite der Dialyseflüssigkeit. Damit kann also über Druckmessungen der TMP bestimmt und während der Behandlung verfolgt werden. Erfindungsgemäß kann der optimale TMP über besagte Druckmessungen bestimmt und über den Dialyseverlauf verfolgt werden. Weicht er zu stark vom Optimum ab, müssen die Flussraten entsprechend nachgeregelt werden. Bei Veränderung der Flussraten von Blut und Dialyseflüssigkeit verändert sich naturgemäß auch die Flussrate an benötigter Substituatflüssigkeit.

In Bezug auf das Substituat ist der Begriff Substitutionsrate entsprechend identisch mit dem Begriff der Flussrate. Wie oben beschrieben wird das Substitutionsziel durch das optimale Gesamtvolumen vorgegeben, welches sich aus der Substitutionsrate in einer bestimmten Zeit ergibt. Über die Druckmessungen auf Blut- und Dialysatseite kann also der optimale Bedarf an Substituatflüssigkeit ermittelt werden, und bei veränderten Bedingungen, die ebenfalls über Druckmessungen ermittelt werden, der Bedarf an Substiatflüssigkeit korrigiert, d.h. das möglicherweise nicht das optimale, jedoch das bestmögliche Substitutionsziel erreicht werden kann.

Unter bestmöglicher Erreichung bzw. Realisierung des Substitutionsziels wird verstanden, dass das vor Behandlung ermittelte oder berechnete Substitutionsziel im Idealfall genau oder in Abhängigkeit von den gegeben Umständen bzw. dem Verlauf der Dialyse möglichst annähernd erreicht wird, wenn die Behandlungszeit als feste Größe vorgegeben wird und daher nicht verändert werden kann. Mit anderen Worten, der entsprechend der ermittelten Filteridentität, Eigenschaften des Filters und der Blutbehandlungseinheit sowie optional aber bevorzugt Patientendaten ermittelte optimale Bedarf an Substituatflüssigkeit sollte genau oder entsprechend der Umstände so genau wie möglich erreicht werden. Dabei ist es bevorzugt, wenn das Substitutionsziel zu 80% erreicht wird, noch bevorzugter zu 85%, noch bevorzugter zu 90%, noch bevorzugter zu 95%, noch bevorzugter zu 96%, noch bevorzugter zu 97%, noch bevorzugter zu 98%, noch bevorzugter zu 99%, und am meisten bevorzugt zu 100% erreicht wird bei fixer Behandlungszeit. Andererseits kann anstelle der Behandlungszeit auch das Substitutionsziel als feste nicht veränderbare Größe definiert werden, welches genau zu erreichen ist bei möglichst geringer Abweichung von der veranschlagten Behandlungszeit, in der Regel bei möglichst geringer Verlängerung der Behandlungszeit.

Die vorliegende Offenbarung betrifft ferner ein Verfahren zur Festlegung des Substitutionsziels bei Blutbehandlungseinheiten, bei dem

I. mindestens zwei Druckmesswerte (PB1, PB2 oder PD1, PD2 oder PB1, PD1 oder PB1, PD2 oder PB2, PD1 oder PB2, PD2) mittels mindestens zweier Drucksensoren ([PB1], [PB2] oder [PD1], [PD2] oder [PB1], [PD1] oder [PB1], [PD2] oder [PB2], [PD1]oder [PB2], [PD2]) zeitgleich oder zeitlich versetzt ermittelt werden, und
II. aus diesen Signalen durch Vergleich mit hinterlegten Daten ein Substitutionsziel für die nachfolgende Dialysesitzung bestimmt wird.

Die Druckmessungen erfolgen mit dem Dialysator und dem Filter der bei der nachfolgenden Dialysesitzung verwendet wird. Zudem erfolgen diese Druckmessungen mit einer Referenzlösung bevor der Patient an die Blutbehandlungseinheit angeschlossen wird.

Bevorzugter Weise kann durch das Verfahren sowohl eine Aussage darüber getroffen werden, wie das optimale Substitutionsziel ist. Zudem kann während der Dialysesitzung erkannt werden, ob Störungen auftreten, so dass das Substitutionsziel und/oder die Dialyseparameter wie Drücke und Flussraten angepasst werden können.

Besonders bevorzugt ist es, wenn mindestens ein Druckmesswert auf der Blutseite und mindestens ein weiteres Druckmesswert auf der Dialysatseite ermittelt werden. Weiter bevorzugt ist es, wenn ein Druckmesswert auf der Blutseite und zwei Druckmesswert auf der Dialysatseite oder zwei Druckmesswert auf der Blutseite und ein Druckmesswert auf der Dialysatseite ermittelt werden. In einer insbesondere bevorzugten Ausführungsform werden zwei Druckmesswerte auf der Blutseite und zwei Druckmesswerte auf der Dialysatseite ermittelt. Es ist jedoch auf möglich und bevorzugt, wenn zwei Druckmesswerte auf der Blutseite gemessen werden, da sich ein Druckpuls nicht nur durch die Filtermembran, sondern auch entlang der Membran fortpflanzt und damit zwei blutseitige Werte auch zur Ermittlung der Filtereigenschaften dienen können. Die Druckmessungen erfolgen dabei an oder unmittelbar vor den Ein- und Auslässen des Tangentialflussfilters TFF hierin auch Dialysator genannt.

Bei den gemessenen Druckmesswerten kann es sich um Absolutdrücke, relative Drücke, absolute Druckdifferenzen zwischen zwei Druckmesspunkten, d.h. Druckmeßsensoren, relative Druckdifferenzen zwischen zwei Druckmeßsensoren, absoluten Druckamplituden, relativen Druckamplituden, Differenzen zwischen den absoluten Druckamplituden an zwei Druckmeßsensoren oder Differenzen zwischen den relativen Druckamplituden an zwei Druckmeßsensoren oder eine Kombination davon oder die Frequenzspektra der Drücke handeln.

Der Begriff "Absolutdruck" oder "Absolutdrücke", wie hierin verwendet, beschreibt den Druck gegenüber dem Atmosphärendruck.

Der Begriff "relativer Druck" oder "relative Drücke", wie hierin verwendet, beschreibt die relative Änderung eines Druckmesswerts in Bezug auf einen zweiten Druckmesswert.

Der Begriff "Druckdifferenz" oder "Druckdifferenzen", wie hierin verwendet, beschreibt die Differenz zweier Drücke.

Der Begriff "Druckamplitude" oder "Druckamplituden", wie hierin verwendet, beschreibt den ermittelten oder gemessenen Wert der Druckschwankungen. Synonym dazu kann der Begriff Druckamplitudenhub verwendet werden.

Der Begriff "Frequenzspektrum" oder "Frequenzspektra", wie hierin verwendet, bezeichnet die Gesamtheit der Frequenzen, die von einem schwingenden System erzeugt werden bzw. in einem Signal enthalten sind.

In einer weiteren bevorzugten Ausführungsform werden die ermittelten Druckmesswerte zu vorgegebenen Referenzwerten oder zu vorher gemessenen Ausgangswerten in Relation gesetzt.

Dabei kann es sich um absolute oder relative Änderungen, Differenzen in den gemessenen Werten, Änderungen in den Druckamplituden oder dem Frequenzspektrum handeln. Die Auswertung kann beinhalten, dass die Änderung von absoluten Werten überwacht wird, insbesondere in Bezug auf ein Unterschreiten oder ein Überschreiten eines Grenzwertes. Dies beinhaltet auch den Vergleich zweier absoluter Werte, deren Differenz zueinander z.B. einen festgelegten Wert nicht über- oder unterschreiten sollte. Das gleiche gilt für relative Änderungen zwischen zwei gemessenen Werten, oder einem gemessenen Wert und einem Referenzwert. Prinzipiell trifft dies auch auf Abweichungen in der Höhe von Amplituden zu bzw. Änderungen im Frequenzspektrum, die sich aus einer Änderung im System ergeben können. Ein exaktes Auswerten des Druckmesswerte, z.B. mittels einer Rechenoperation, kann auch ein Abschätzen des Verlaufs des Druckmesswerts beziehungsweise eines Trends des Druckmesswerts bzw. der Höhe oder des Verlaufs beinhalten. Für die Auswertung des Druckmesswerts kann erfindungsgemäß jede aus dem Signal erkennbare oder ableitbare Information sowie aus den Mess- oder Bestimmungsbedingungen ableitbare Information verwendet werden. So kann z.B. die Höhe der Amplitude, Veränderungen in der komplexen Amplitude der Frequenzen und damit auch in den harmonischen Frequenzen, relative Änderungen zweier Druckmesswerte zueinander oder Verschiebungen in den absoluten Werten von Druckmesswerten eine Aussage erlauben. Dies umfasst unter anderem auch den Vergleich von Druckmesswerten unterschiedlicher Patienten oder Systemen oder Behandlungsmethoden untereinander. Derartige Daten und das Ziehen von Erkenntnissen hieraus kann ebenfalls ein Auswerten im Sinne der Erfindung sein.

Dabei hat sich überraschend herausgestellt, dass die Messung und Auswertung der Druckmesswerte, z.B. erzeugt auch durch eine Blutpumpe P im System, Aufschluss über die Flusseigenschaften in einer Blutbehandlungseinheit gibt. Aus diesen Druckmesssignalen an einer Blutbehandlungseinheit kann ein optimales Substitutionsziel während einer Dialyse ermittelt werden. Wenn im Verlauf der Dialysesitzung Störungen auftreten, kann gegebenenfalls nachgeregelt werden. Erfindungsgemäß kann jeder Druckmesswert unabhängig seines Ursprungs zur Auswertung benutzt werden. Beispielsweise werden in einer Ausführungsform die Druckmesswerte, die durch das Schalten einer Bilanzkammer BK entstehen, ermittelt und für die Auswertung genutzt.

Die Überwachung der Systemeigenschaften oder der Dialyseparameter während der Behandlung wird vorzugsweise derart umgesetzt, dass die durch die Blutpumpe P erzeugten Drucksignale und deren Propagation überwacht werden. Erfindungsgemäß können auch die Drucksignale einer dialysatseitigen Pumpe oder die durch das Schalten von Ventilen erzeugten Druckspitzen überwacht werden. Es ist auch möglich, dass kombinierte Drucksignal aus beiden Pumpen zu überwachen, oder jedes Drucksignal einzeln zu erfassen. Insbesondere bevorzugt ist die Überwachung von den 4 Drucksignalen an den Eingängen und Ausgängen des Tangentialflussfilters TFF.

Je nach den Systemeigenschaften der Blutbehandlungseinheit breiten sich die Drucksignale in dem System entlang der Blutflussrichtung und in Transmembranrichtung aus.

Es ist also möglich, durch die Überwachung der Drucksignale auf der Blutseite und der Dialysatseite sowie durch die Überwachung der Verhältnisse dieser Drücke, Änderungen der Flussbedingungen zu registrieren und das Substitutionsziel dementsprechend anzupassen.

Der Begriff "*Blutbehandlungseinheit*" bezieht sich auf eine Vorrichtung zur Behandlung von Blut, und ganz besonders auf eine Vorrichtung zur extrakorporalen Blutbehandlung. Der Begriff beschreibt dabei eine Vorrichtung, die zur Reinigung und/oder Behandlung von Blut eingesetzt werden kann. Insbesondere kann es sich um eine Dialysevorrichtung handeln, die zur Hämodialyse, Hämoperfusion, Hämofiltration oder Hämodiafiltration in der Lage ist.

Der Begriff "*Systemänderung*", wie hierin verwendet, umfasst die Interaktion von Bestandteilen der Vorrichtung zur Behandlung von Blut, d.h. der Blutbehandlungseinheit, insbesondere der Filtermembran mit Blut. Durch diese Wechselwirkung verschlechtern sich die Flusseigenschaften sowohl in Transmembranrichtung als auch in Blutflussrichtung. Dies wird beispielsweise durch Thrombozytenanlagerung, Koagelbildung ("Clotting"), chemisches Binden von Blutbestandteilen an die Membran oder schlicht mechanisches (strömungsbedingtes) Drücken der Blutbestandteile an und sogar in die Membran verursacht, ist aber nicht auf diese beschränkt und kann auch an anderen Stellen innerhalb der Blutbehandlungseinheit auftreten. Zusätzlich kann eine Systemänderung durch beispielsweise einen abgeknickten Schlauch, undichte Stellen oder einen gelösten Anschluss erkannt werden.

Die synonymen Begriffe "*Systemeigenschaften*" oder "*Dialyseparameter*" bezeichnen die Einstellungen der Blutbehandlungseinheit in Bezug auf die Drücke und Flussraten von Blut, Dialysat und Substituat.

Das Verfahren umfasst weiterhin die Analyse und Charakterisierung der Flusseigenschaften in einer Vorrichtung zur Behandlung von Blut zur Bestimmung der Filtereigenschaften, durch die folgenden Schritte:

a) Bestimmung der Filtereigenschaften durch Ermittlung von mindestens zwei Druckmesswerten an mindestens zwei Drucksensoren innerhalb der Blutbehandlungseinheit mit einer Referenzlösung,
b) optional Vergleich der gemessenen Druckmesswerte mit auf der Speichereinheit hinterlegten Referenzwerten desselben Patienten, welche in früheren Dialysesitzungen erhoben wurden oder mit auf der Speichereinheit hinterlegten Referenzwerten einer Patientengruppe,
c) Berechnung eines Substitutionsziels auf Grundlage der Werte gemäß Schritt a) und optional Schritt b).

Dieses Verfahren ist vorteilhaft, weil vor der Blutbehandlung, d.h. ohne Patienten, durch Aufnahme von Druckmesswerten mit einer Referenzlösung die Eigenschaften des verwendeten Filters ermittelt werden können. Sind die Filtereigenschaften eines konkreten Filtertyps auf der Speichereinheit hinterlegt, so können nicht nur die Filtereigenschaften sondern sogar der konkrete Filtertyp ermittelt werden.

Dies ist ein großer Vorteil für den Patienten, weil heutzutage Transmembranfilter Massenwaren sind und solche Dialysatoren von den Dialysestationen von den gerade preiswertesten Anbietern bezogen werden und ein Patient daher bei verschiedenen Dialysesitzungen verschiedene Dialysatoren erhält. Zudem ist es zu aufwendig, das Substitutionsziel per Hand bei jeder Dialysesitzung für jeden Patienten wieder neu zu optimieren, so dass oftmals nur Standardeinstellungen verwendet werden, welche für den Patienten jedoch in der Regel nicht das am besten geeignete Substitutionsziel bedeuten. Dieses Problem löst die vorliegende Erfindung zum einen durch Bestimmung der Filtereigenschaften und eines daraus berechneten Substitutionsziels, welches zudem noch mit vorhandenen Patientendaten abgeglichen werden kann. Sind die Patientendaten oder Daten einer ähnlichen Patentengruppe auf der Blutbehandlungseinheit hinterlegt, so erfolgt die Berechnung des für den Patienten optimalen Substitutionsziels mit vorheriger Bestimmung der Filtereigenschaften vollautomatisch. Die Berechnung eines optimalen am Ende der Dialysesitzung zu erreichenden Substitutionsziels macht nur dann wirklich Sinn, wenn nach einer solchen Berechnung auch gewährleistet wird, dass dieses Substitutionsziel am Ende der Dialysesitzung auch erreicht wird. Genau dies ermöglicht die erfindungsgemäße Blutbehandlungseinheit.

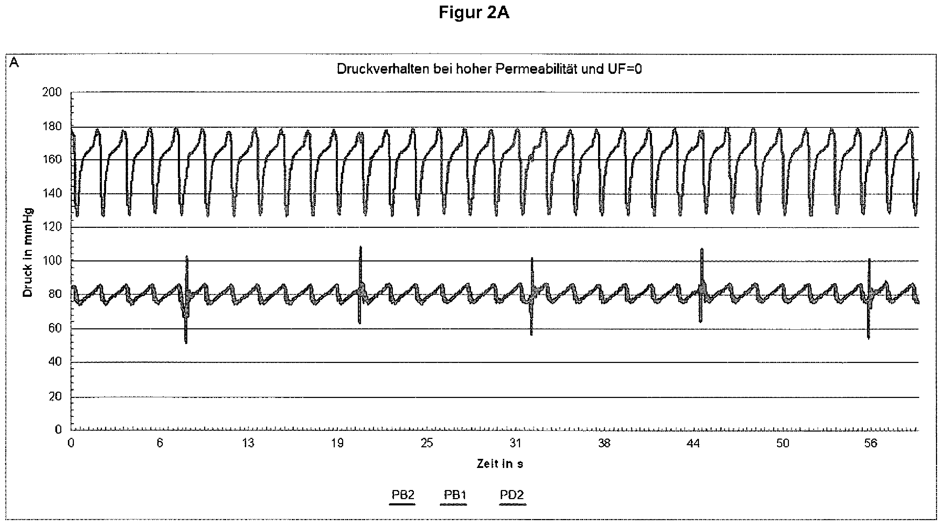

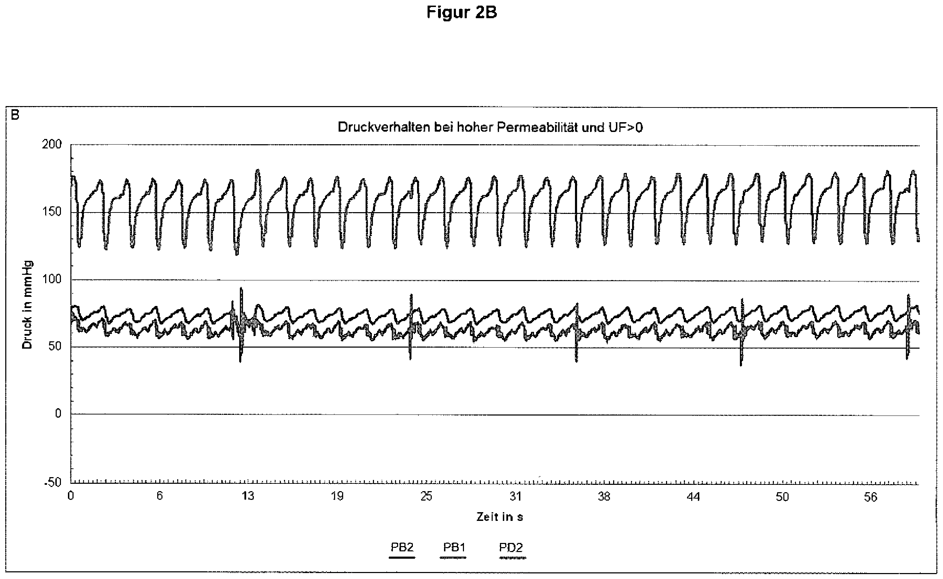

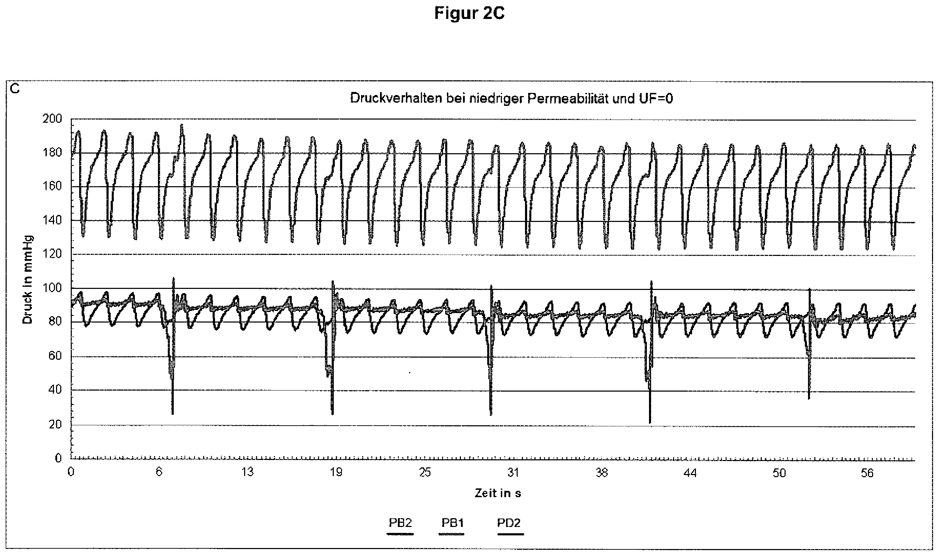

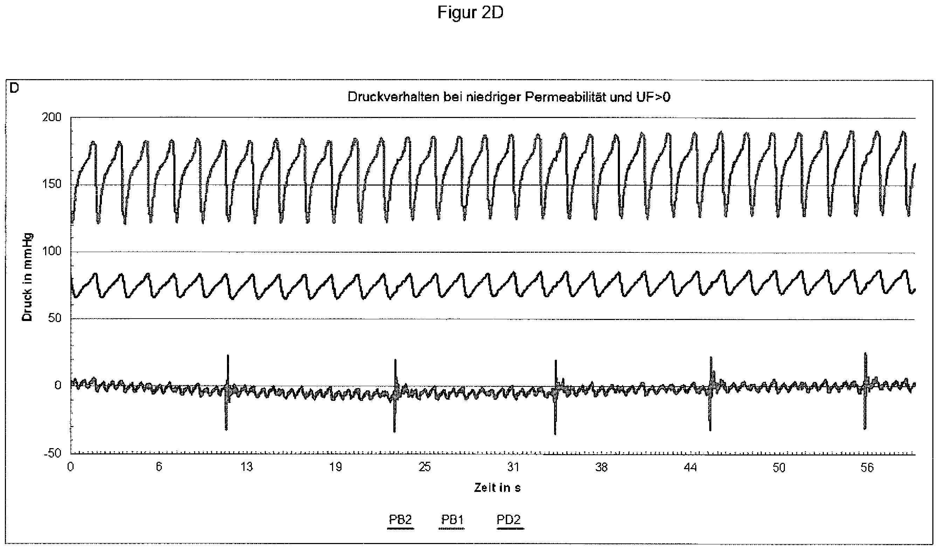

Die Filtereigenschaften können beispielsweise hinsichtlich ihrer Unterschiede besonders in Bezug auf die Permeabilität durch Druckmessungen bestimmt werden (Figur 2). Besonders der Pulsübertrag und dialysatseitiges Druckniveau bzw. TMP können bei einer Messung mit der Referenzlösung Aufschluss geben, ob ein Filter für eine HDF Therapie geeignet ist.

Bei Filtern mit geringer Permeabilität ist beispielsweise der Übertrag im Prinzip nicht vorhanden, d.h. hier breiten sich die Pulse nur entlang des Blutflusses von PB1 nach PB2 aus, d.h. die Signale werden im Prinzip nur im blutseitigen Kreislauf erfasst (Figur 2C). Bei einem Filter mit hoher Permeabilität breitet sich der Puls auch über die Membran auf die Dialysatseite aus, wobei die Signale gleichstark sein können (Figur 2A), in der Regel aber eher unterschiedlich stark sind. Dabei wird bei einem stärkeren Übertrag durch die Membran der Übertrag entlang des Blutflusses schwächer.

Wird weiterhin auf der Dialysatseite die Leistung der Ultrafiltrationspumpe hochgeregelt, verschiebt sich zusätzlich das dialysatseitige Druckniveau; bei einem low-flux-Filter ist diese Verschiebung viel stärker (Figur 2D) als bei einem high-flux-Filter (Figur 2B), weil mehr Energie aufgewendet werden muss, um den gleichen transmembranen Fluss zu erzeugen.

In Tabelle 1 sind zwei Filter mit unterschiedlicher Faseranzahl gegenübergestellt. Die Filter unterscheiden sich nur in der Anzahl der Fasern, was auf die unterschiedlichen Membranoberflächen zurückzuführen ist. Fasergeometrie und Permeabilität sind sonst identisch.

Betrachtet wird hier die Druckamplitude in PB1, PB2 und PD2 sowie die relative Amplitude jeweils bezüglich PBE. Entscheidend ist, dass der größere Filter, der somit besser für die HDF Therapie mit hohen Volumina geeignet ist, einen besseren Impulsüberträg auf die Dialysatseite (PD2) gewährleistet. Somit ist dieser auch besser für hochvolumige Substitution geeignet.

## Tabelle 1

| | high flux Filter mit 1,8 m² effektiver Filteroberfläche | | | | |
|---|---|---|---|---|---|
| BF in ml/min | PB1 Mittel | PB2 Mittel | PD2 Mittel | PB2/PB1 | PD2/PB1 |
| 100 | 27,06 | 8,63 | 12,99 | 31,9% | 48,0% |
| 200 | 52,97 | 12,17 | 18,03 | 23,0% | 34,0% |
| 300 | 87,20 | 16,12 | 26,90 | 18,5% | 30,8% |

| | high flux Filter mit 2,3 m² effektiver Filteroberfläche | | | | |
|---|---|---|---|---|---|
| BF in ml/min | PB1 Mittel | PB2 Mittel | PD2 Mittel | PB2/PB1 | PD2/PB1 |
| 100 | 22,61 | 8,12 | 12,86 | 35,9% | 56,9% |
| 200 | 45,45 | 11,32 | 18,29 | 24,9% | 40,2% |
| 300 | 79,65 | 15,85 | 28,98 | 19,9% | 36,4% |

Durch das hinterlegen solcher Daten sowie die verwendete Referenzlösung (in diesem Fall physiologische NaCl-Lösung) können Filter nicht nur relativ zu einander verglichen werden, sondern auch eindeutig identifiziert werden, falls Vergleichswerte auf der Blutbehandlungseinheit hinterlegt sind.

Durch die in Schritt a) durch die ermittelten Druckmesswerte bestimmten Filtereigenschaften kann bei einem bekannten Filter oder auch einem unbekannten Filter der zu erwartende Verlauf der folgenden Blutbehandlung vorherbestimmt werden. Das ist vorteilhaft, weil so vor Beginn der Dialyse ohne Bedarf an einem Probelauf mit Patientenblut das entsprechend der Filtereigenschaften ermittelte optimale Volumen an benötigter Substitutionsflüssigkeit, als das Substitutionsziel, ermittelt werden kann. Dieser Schritt ist auch deshalb vorteilhaft, weil sich auch einzelne Filter desselben Typs hinsichtlich ihrer filterspezifischen Eigenschaften unterscheiden können und zu unterschiedlichen Dialyse-Verläufen führen können. Weiterhin ist dieser Schritt auch vorteilhaft, weil bei Wiederverwendung desselben Filters selbst bei gründlicher Reinigung des Filters wesentliche Eigenschaften verändert sein können, wobei eine Wiederverwendung zur Zeit nur in den USA praktiziert wird.

Dabei umfasst Schritt a) vorzugsweise die Einzelschritte

a1) Ermittlung von Druckmesswerten innerhalb der Blutbehandlungseinheit mit einer Referenzlösung, und

a2) Bestimmung der Filtereigenschaften durch Vergleich der in Schritt a1) gemessenen Druckmesswerte mit auf der Speichereinheit hinterlegten Druckmesswerten.

Schritt a2) kann auch durch Vergleich mit auf der Speichereinheit hinterlegten Druckmessbereichen konkreter Filtertypen durchgeführt werden, um nicht nur die Filtereigenschaften sondern auch den konkreten Filtertyp zu bestimmen. Sind keine Druckmesswerte von konkreten Filtertypen hinterlegt, so werden wir gemäß Schritt a1) ermittelten Druckmesswerte mit hinterlegten Druckmesswerten vergleichen, welche die Filtereigenschaften repräsentieren, so dass aus diesem Vergleich die Filtereigenschaften ermittelt werden können.

Die in Schritt a) ermittelten Druckmesswerte können auch zur Identifizierung eines bestimmten Filtertyps dienen, wenn die filterspezifischen Eigenschaften auf der Speichereinheit der Blutbehandlungseinheit hinterlegt bzw. abgespeichert worden sind. Ein Dialysefilter wird durch filtertypische Eigenschaften wie Gesamtgröße, Anzahl der Hohlfasern oder -röhren, Oberflächengröße und Siebkoeffizient charakterisiert. Diese Charakteristika bedingen ein bestimmtes Strömungsverhalten der Referenzflüssigkeit, welches durch definierte Druckmesswerte erkannt wird. Dementsprechend kann der Filter identifiziert werden. Diese Identifikation ist vorteilhaft, weil dadurch empirisch ermittelte filterspezifische Werte genutzt werden können, die in Behandlungen ermittelt wurden, die besonders gut verlaufen sind. Damit kann das Substitutionsziel auf Grund des ermittelten Filtertyps ermittelt werden. Das Verfahren umfasst dann folgende Schritte:

a) Bestimmen der Filtereigenschaften durch Ermittlung von mindestens zwei Druckmesswerten an mindestens zwei Drucksensoren innerhalb der Blutbehandlungseinheit mit einer Referenzlösung und Vergleich mit auf der Speichereinheit hinterlegten filterbezogenen Druckmesswerten;
b) optional Vergleich der in Schritt a) gemessenen Druckmesswerte mit auf der Speichereinheit hinterlegten Referenzmesswerte desselben Patienten, welche in früheren Dialysesitzungen erhoben wurden oder mit auf der Speichereinheit hinterlegten Referenzwerten einer Patientengruppe,;
c) Berechnung des Substitutionsziels auf der Grundlage der Werte gemäß a) und optional b);

Bei den Referenzwerten einer Patientengruppe handelt es sich vorzugsweise um eine dem zu behandelnden Patienten ähnliche Patientengruppe, so dass sich aus dieser ähnlichen Patientengruppe ein gemittelter Wert ergibt, der auch für die zu behandelnden Patienten anwendbar ist.

Durch Vergleich der gemessenen Werte in Schritt b) mit auf der Speichereinheit hinterlegten Patientendaten, d.h. durch Vergleich mit Werten aus besonders gut verlaufenden Behandlungen können damit entsprechend verwendetem Filter und behandeltem Patient oder mit Bezug auf eine Patientengruppe das optimale Substitutionsziel ermittelt werden. Dabei kann auf Werte ein und desselben Patienten aus einer einzigen gut bis optimal verlaufenden Behandlung zurückgegriffen werden, vorzugsweise jedoch auf gemittelte Werte aus möglichst gut verlaufenden Behandlungen. Gemittelte Werte werden hierbei als Durchschnittswerte der Behandlungsverläufe verstanden. Die gemittelten Werte bilden damit einen Erwartungswert für die weitere bzw. kommende Behandlung. Vorzugsweise sind die Daten aus vorherigen Dialyseverläufen auch gewichtet, d.h. dass bestimmte Dialyseverläufe hinsichtlich ihrer Wichtigkeit, Bedeutung oder unter Berücksichtigung von örtlichen und zeitlichen Unterschieden, wie zum Beispiel Filtertyp oder Blutbehandlungseinheit bzw. Dauer der Dialyse unterschiedlich bewertet werden.

Bei Vergleichen mit hinterlegten Werten von bestimmten Patientengruppen wird auf Werte gleichartiger Patienten zurückgegriffen. Unter gleichartigen Patienten werden Patienten mit ähnlichen Blutwerten, ähnlicher Nierendysfunktion, etwa dem gleichen Alter, Gewicht und Geschlecht sowie ähnlich Behandlungshistorie verstanden. Hier werden die Daten gemittelt und bevorzugt auch gewichtet.

Dementsprechend kann das optimale Substitutionsziel wie oben beschrieben festgelegt werden.

Die Ermittlung der Druckmesswerte erfolgt über Drucksensoren. Hierfür können die aus dem Stand der Technik bekannten Drucksensoren benutzt werden, wie z.B. piezoresistive, piezoelektrische, frequenzanaloge, Drucksensoren mit Hallelementen, kapazitive, induktive und/oder Kombinationen davon. Bevorzugt sind hierbei Drucksensoren, deren Abtastrate vorzugsweise mindestens 20 Hz beträgt. Die Abtastrate bezeichnet hierbei die Rate mit der Signalwerte aus einem kontinuierlichen Signal entnommen werden.

Die ermittelten Druckmessdaten können zur Klassifikation des Filtrationspotentials der Blutbehandlungseinheit herangezogen werden. Hierzu werden die Messwerte den entsprechenden auf der Speichereinheit hinterlegten Klassifikationsgruppen zugeordnet. Jeder Klassifikationsgruppe entspricht dann eine Handlungsanweisung, wie gegebenenfalls das Substitutionsziel anzupassen ist. Eine solche Handlungsanweisung kann über eine Anzeige dem Bedienpersonal vorgeschlagen werden oder aber automatisch am Dialysegerät umgesetzt werden.

Alternativ dazu kann einem bestimmten gemessenen Filtrationspotential ein definierter Ultrafiltrationswert zugeordnet werden.

Erfindungsgemäß wird mindestens ein Drucksensor, bevorzugter zwei Drucksensoren, noch bevorzugter drei Drucksensoren und am meisten bevorzugt vier Drucksensoren eingesetzt. Erfindungsgemäß können bei Bedarf auch weitere Drucksensoren eingesetzt werden.

Die erfindungsgemäßen Zusammenhänge präsentieren sich im Detail folgendermaßen:
Die transmembranöse Ausgangssituation kann erfasst werden, indem das Verhältnis der Druckmesswerte, z.B. das der Amplituden ($A_i$), an den Drucksensoren [PD2] und [PB1] ermittelt wird. Dieser Parameter wird überwacht und optional wird bei Unterschreiten oder Überschreiten eines definierten Wertes (einmalig oder über einen definierten Zeitraum) eine Gegenmaßnahme vorgeschlagen bzw. automatisch eingeleitet.

Eine sinkende Permeabilität der Filtermembran drückt sich einerseits in einer Verkleinerung des Verhältnisses aus $A_{PD2}/A_{PB1}$ aus und andererseits wird auf Grund der Impulserhaltung auch eine Steigerung von $A_{PB2}/A_{PB1}$ verursacht.

Der blutseitige Flusswiderstand kann aus der Differenz der gemessenen Druckmesswerte aus PB1 und PB2 und/oder dem Verhältnis aus PB1 und PB2 und/oder der Änderung der Druckamplitude $A_{PB1}$ und/oder die Änderung der Druckamplitude $A_{PB2}$ und/oder das Verhältnis der Druckamplituden $A_{PB1}$ und $A_{PB2}$ erfasst werden und dient als Indikator für den blutseitigen Flusswiderstand. Einer oder mehrere dieser Parameter werden überwacht und bei Unterschreiten oder Überschreiten eines definierten Wertes kann eine Gegenmaßnahme vorgeschlagen bzw. automatisch eingeleitet.

Eine Verengung im blutseitigen Kreislauf, z.B. durch Koagelbildung im Filter oder im Patientenrücklauf, führt je nach Lage der Verengung zu einer charakteristischen Verschiebung der Verhältnisse aus PB1 und PB2. Liegt sie beispielsweise zwischen den beiden Messpunkten, so erhöht sich die Differenz dieser beiden Druckwerte, liegt sie dahinter, so tritt ein paralleles Anheben beider Werte auf.

Der transmembranöse Flusswiderstand ermittelt sich aus der Differenz der gemessenen Druckmesswerte aus PB1 und PD2 und/oder dem Verhältnis aus PB1 und PD2 und/oder der Differenz aus PB2 und PD2 und/oder dem Verhältnis aus PD2 und PB2. Weiterhin dienen Änderungen der Druckamplitude $A_{PD2}$ und/oder dem Verhältnis der Druckamplituden $A_{PB1}$ und $A_{PD2}$ und/oder dem Verhältnis der Druckamplituden $A_{PB2}$ und $A_{PD2}$ und/oder der Differenz aus PB1 und PD1 und/oder dem Verhältnis aus PB1 und PD1 und/oder der Differenz aus PB2 und PD1 und/oder dem Verhältnis aus PB2 und PD1 und/oder der Änderung der Druckamplitude $A_{PD1}$ und/oder dem Verhältnis der Druckamplituden $A_{PB1}$ und $A_{PD1}$ und/oder dem Verhältnis der Druckamplituden $A_{PB2}$ und $A_{PD1}$ als Indikator für den transmembranen Flusswiderstand.

Gleichermaßen können $A_{PB1}$, $A_{PD1}$ oder $A_{PD2}$ oder einer Kombination davon als Indikator für die Permeabilität der Filtermembran und Filteroberfläche verwendet werden.

Desweiteren können PB1 und PB2 oder die Druckamplituden von PB1 und PB2 als Indikator für den Flusswiderstand des Filters verwendet werden.

Dem entspricht, dass die ermittelten Druckmessdaten zur Berechnung der Permeabilität, der durchlässigen Oberfläche und des Flusswiderstands des Filters verwendet werden können.

Eine Konsequenz kann beispielsweise sein, dass bei einem erhöhten Widerstand in Blutflussrichtung und dem damit verbundenen Erreichen der Grenzwerte blutseitiger Druckmesswerte eine Spülung mit einer Kochsalzlösung, eine Erhöhung der Spülrate, eine stärkere Blutverdünnung oder eine Änderung des Blutflusses oder eine Kombination davon vorgeschlagen oder eingeleitet wird.

Kommt es zu einem Zusammenfall eines erhöhten blutseitigen Flusswiderstands mit einer Erhöhung des Hämatokrits des Patienten kann beispielsweise die Erhöhung der Dosierung des blutverdünnenden oder antikoagulativen Mittels vorgeschlagen oder eingeleitet werden. Im umgekehrten Fall wie einem Zusammenfall eines erniedrigten blutseitigen Flusswiderstands mit einem geringen Hämatokrit des Patienten kann die Erniedrigung der Dosierung des blutverdünnenden oder antikoagulativen Mittels vorgeschlagen oder eingeleitet werden.

Mit [PB1] wird der Drucksensor im Blutkreislauf vor dem Bluteinlass in den Tangentialflussfilters TFF bezeichnet und mit PB1 der am Drucksensor [PB1] gemessene Druck. Mit [PB2] wird der Drucksensor im Blutkreislauf nach dem Blutauslass aus dem Tangentialflussfilters TFF bezeichnet und mit PB2 der am Drucksensor [PB2] gemessene Druck. Mit [PD1] wird der Drucksensor im Dialysatkreislauf vor dem Dialysateinlass in den Tangentialflussfilters TFF bezeichnet und mit PD1 der am Drucksensor [PD1] gemessene Druck. Mit [PD2] wird der Drucksensor im Dialysatkreislauf nach dem Dialysatauslass aus dem Tangentialflussfilters TFF bezeichnet und mit PD2 der am Drucksensor [PD2] gemessene Druck.

Bisherige Lösungsansätze konnten nicht zwischen Veränderungen in Blutflussrichtung, Dialysatflussrichtung und Transmembran-Richtung differenzieren, wodurch eine gezielte Behebung von Störungen nicht möglich war. Durch die Verwendung von bis zu vier Drucksensoren und der Auswertung der erfassten Druckmesswerte an jedem dieser Drucksensoren, können alle oben genannten Verhältnisse überwacht werden. Aus der Gesamtheit der Druckmesswerte kann eine vollständige Auswertung erfolgen, die es ermöglicht, die Art der Systemänderung gezielt zu bestimmen. Durch eine geeignete Auswertung von vier Druckmesswerten läßt sich auch die Lage der Störung zumindest eingrenzen.

Bevorzugt werden die Druckmesswerte blutseitig am Drucksensor [PB1] sowie am Drucksensor [PB2] ermittelt. Dialysatseitig werden die Druckmesswerte bevorzugt am Drucksensor [PD2] und am Drucksensor [PD1] ermittelt.

In einer alternativen Ausführungsform ist es jedoch ausreichend, wenn die Druckmesswerte PB1 und PB2 und eines der dialysatseitigen Signale PD1 oder PD2 vorliegt.

Desweiteren kann der Druck PD1 oder PD2 oder beide zusammen während des transmembranösen Spülens in Abhängigkeit von der Geschwindigkeit des Spülflusses beobachtet werden.

Die Auswertung erfolgt durch den Vergleich von mindestens zwei oder mehr Druckmesswerten, derart, dass es sich hierbei um die Absolutdrücke und/oder die relativen Drücke und/oder die Druckdifferenzen und/oder die Druckamplituden und/oder die Differenz der Druckamplituden und/oder die relativen Druckamplituden und/oder die Frequenzspektra handelt. Für die Auswertung werden Änderungen der gemessenen Druckmesswerte zu vorgegebenen Referenzwerten und/oder Änderungen zu vorher gemessenen Ausgangswerten erfasst. Dabei kann es sich um absolute und/oder relative Änderungen, Differenzen in den gemessenen Werten, Änderungen in den Druckamplituden und/oder dem Frequenzspektrum handeln. Auswertbar sind hierbei alle Informationen, die sich aus den ermittelten Daten entnehmen lassen, insbesondere die Höhe der Amplituden, gemittelte Werte, Trends, Integrale, Differentiale, Verzögerungen von Druckschwankungen, Fluktuationen, Kopplungsgrad oder Korrelationsgrad, Verteilung der Signale etc..

Die Auswertung der ermittelten Druckmesswerte erfolgt über Vorrichtungen, die dem Fachmann aus dem Stand der Technik bekannt sind, wobei es sich bei den Vorrichtungen bevorzugt um eine Speichereinheit und eine zentrale Recheneinheit handelt. Die zentrale Recheneinheit kann zum Auswerten der Messdaten z.B. eine CPU umfassen, welche Änderungen der gemessenen Druckmesswerte zu vorgegebenen Referenzwerten und/oder Änderungen zu vorher gemessenen Ausgangswerten berechnet. Die Auswertung kann in Form von absoluten und/oder relativen Änderungen, Differenzen in den gemessenen Werten, Änderungen in den Druckamplituden, z.B. der Höhe der Amplituden und/oder dem Frequenzspektrum erfolgen.

Ergeben die gemessenen Druckmesswerte an den mindestens zwei Drucksensoren ausgewählt aus der Gruppe bestehend aus [PB1], [PB2], [PD1] und [PD2], dass die gemessenen Druckmesswerte über ein Toleranzintervall hinaus von den Referenzdruckmesswerten abweichen, so folgt daraus, dass die Dialyseparameter, nämlich die Flußraten von Blut, Dialysat und/oder Substituat angepasst werden müssen. Bei den Referenzdruckmesswerten handelt es sich um Druckmesswerte, welche bei Dialysesitzungen erhalten wurden, bei denen das Substitutionsziel erreicht worden ist. Vergleicht man daher die gemessenen Druckmesswerte mit den hinterlegten Referenzdruckmesswerten unter Berücksichtigung eines eventuell anderen Transmembranfilters und/oder einer eventuell veränderten Patientenkonstitution, so lässt sich aus dem Vergleich vorhersagen, ob das berechnete Substitutionsziel erreicht werden wird.

Unter Anpassung der Flussrate von Blut, Dialysat und/oder Substituat wird eine Adaption an geänderte Bedingungen innerhalb der Blutbehandlungseinheit verstanden, um dem berechneten Substitutionsziel am nächsten zu kommen, wenn die Behandlungszeit festgelegt ist oder die vorgegebene Behandlungszeit möglichst wenig zu verändern, wenn das Substitutionsziel festgelegt ist. Dabei ist es bevorzugt, wenn die entsprechenden vorgenommenen Änderungen nicht sprunghaft erfolgen, sondern langsam oder auch verzögert. Diese allmähliche Anpassung ist vorteilhaft, weil sich langsame Steigerungen der Substitutionsrate beispielsweise vorteilhaft auf die Ausbildung der Sekundärmembran auswirken. Durch zu abrupte Flussratenänderungen könnten außerdem Störungen im System entstehen. Dabei kann die Anpassung bevorzugt durch eine langsame Steigerung oder auch Senkung der Blutflussrate oder der Dialyseflüssigkeitsflussrate geschehen. Jedoch führt eine Steigerung nicht notwendigerweise immer zu einer besseren Erreichung des Substitutionsziels; bei einer Erhöhung über einen festgelegten Grenzwert hinaus kann sich zum Beispiel der Transmembrandruck der Filtermembran verringern und damit die Filtrationseffizienz sinken. Darum ist es auch bevorzugt, die Flussraten ggf. zu verringern.

Die Anpassung kann automatisch durch das System erfolgen, wenn ein entsprechendes Steuerprogramm auf der zentralen Recheneinheit hinterlegt ist. Die über die Drucksensoren erfassten Änderungen werden mit hinterlegten Werten abgeglichen und Steuerbefehle zur Anpassung von regelbaren Systemparametern, z.B. der Flussraten gegeben, um das berechnete Substitutionsziel bestmöglich zu erreichen. Dabei ist es bevorzugt, wenn das System bei Regelschritten zur Anpassung an geänderte Bedingungen ein akustisches Signal über Lautsprecher und/oder visuelles Signal über den Bildschirm ausgibt.

Es ist weiterhin bevorzugt, dass ein weiteres Signal ertönt, wenn innerhalb einer definierten Zeitspanne mehrfach eine Anpassung durchgeführt wurde. Dieses Warnsignal ertönt bevorzugt bei mehr als drei Anpassungen innerhalb einer Stunde, noch bevorzugter innerhalb einer halben Stunde, und noch bevorzugter innerhalb von 10 Minuten. Das Erteilen dieses Warnsignals ist vorteilhaft, weil die dichte Folge notwendiger Anpassungen auf Systemänderungen wie weiter oben ausgeführt sein kann, beispielsweise geknickte Schläuche oder Verstopfungen oder undichte Stellen im System hindeuten können. Zur Überprüfung und Behebung der Störungen wäre hier das Eingreifen von geschultem Personal oder Fachleuten notwendig. Liegen solche außerplanmäßigen Störungen der Blutbehandlungseinheit jedoch nicht vor, so können notwendige Anpassungen der Flußraten angezeigt oder auch von der Blutbehandlungseinheit automatisch vorgenommen werden, um das Substitutionsziel bestmöglichst innerhalb der vorgegebenen Behandlungszeit zu erreichen bzw. von der veranschlagten Behandlungszeit möglichst wenig abzuweichen, insbesondere diese möglichst wenig zu verlängern, wenn das Substitutionsziel als fixe Größe festgelegt ist.

Die Anpassung kann auf der Basis von über den Bildschirm ausgegeben Werten auch von geschultem Personal erfolgen. Auch hier gelten die oben genannten Bedingungen zur allmählichen Anpassung.

In einer bevorzugten Ausführungsform erfolgt die Auswertung der Druckmesswerte an einer zentralen Recheneinheit. Diese zentrale Recheneinheit umfasst eine bevorzugt eine CPU, einen Eingang für die Druckmesswerte und eine Anzeige für die Druckmesswerte und/oder die ermittelten Handlungsempfehlungen. In einer besonders bevorzugten Ausführungsform enthält die zentrale Recheneinheit zusätzlich einen Ausgang, über den das ermittelte, an die Störungen angepasste Substitutionsziel automatisch an das Dialysegerät weitergeleitet wird und am Dialysegerät automatisch die ermittelte Anpassung durchgeführt wird.

Die Effektivität der Blutbehandlung ist primär abhängig von vier Faktoren: der Behandlungszeit, dem Blutfluss, die Clearance und dem Dialysatfluss. Besonders die ausreichend lange Behandlungszeit muss gewährleistet sein und ist ein Hauptfaktor für eine erfolgreiche Behandlung. Zahlreiche Studien haben ergeben, je höher die verabreichte Dialysedosis, desto niedriger ist (über einen breiten Korrelationsbereich) auch die Patientensterblichkeit.

Durch das behandelnde Personal zu behebende Störungen im Betrieb akkumulieren sich schnell zu mehreren Vorfällen pro Jahr. Im Extremfall müssen Sitzungen sogar abgebrochen werden. Weitaus häufiger sind jedoch die Fälle, bei denen eine Störung nicht erkannt und daher auch nicht behoben wird, was zu einem suboptimalen Dialyseergebnis führt. Durch eine gezielte Behebung von Störungen, werden diese Auszeiten auf ein Minimum reduziert und können in manchen Fällen auch komplett vermieden werden, indem die richtige Maßnahme während des laufenden Betriebes eingeleitet wird. Die Suche nach dem Grund der Störung entfällt weitestgehend und gibt damit auch dem Patienten ein erhöhtes Gefühl der Sicherheit. Infolge dessen wird die Dialyseeffizienz gesteigert und auch die Wirtschaftlichkeit der Dialyse erhöht.

Die während der Behandlung ermittelten Druckmesswerte werden über die zentrale Recheneinheit mit den zu Beginn über die Referenzlösung ermittelten Referenzdruckmesswerten und Referenzdruckmesswerten, die während anderer Behandlung ermittelt wurden verglichen. Bei der Abweichung von mindestens einem gemessenen Wert von dem entsprechenden Referenzdruckmesswert erfolgt eine Anpassung der Flussraten. Diese Anpassung kann durch die Veränderung, also Erhöhung oder Verringerung, der Flussraten von Blut, Dialyseflüssigkeit oder Substituatflüssigkeit erfolgen, besonders bevorzugt aber der Substituatflüssigkeit (hierin auch nur als Substituat bezeichnet). Die Behandlungsdauer bleibt dabei gleich. Es ist also bevorzugt, wenn das bei festgelegter Behandlungsdauer die Flussraten so verändert werden, dass das festgelegte Substitutionsziel bestmöglich realisiert werden kann.

Es ist weiterhin auch bevorzugt, wenn das festgelegte Substitutionsziel erreicht wird, indem die Behandlungsdauer verändert, bevorzugt verlängert, wird und die Flussraten gleich bleiben, wobei es besonders bevorzugt ist, wenn die Behandlungsdauer möglichst wenig verändert wird.

Der Begriff "*Flusseigenschaften*", wie hierin verwendet, bezieht sich auf die Gesamtheit der Eigenschaften der jeweils fließenden Flüssigkeit. Von besonderem Interesse sind die dynamische Viskosität, die Flussgeschwindigkeit, das Flussvolumen, das Strömungsprofil, osmotischer Druck, die Oberflächenspannung sowie die von den verwendeten Pumpen sowie den übrigen aktiven Betriebselementen wie elektrischen Vorrichtungen und passiven Betriebselementen wie dem Schlauchsystem und dem Dialysator erzeugten Veränderungen und Artefakte.

Der Begriff "*Verhältnis*", wie hierin verwendet, ist nicht zwingend auf den Quotienten aus zwei Größen beschränkt, sondern kann auch die Differenz oder jegliche andere Kennzahl, mit der sich das "*Verhältnis*" zwischen zwei Größen ausdrücken lässt, umfassen.

In einer Ausführungsform ist die relative Änderung einzelner Messpunkte über die Zeit $A_{PDi}(t)/A_{PDi}(t=0)$ bzw. $A_{PBi}(t)/A_{PBi}(t=0)$ ein geeigneter Kontrollparameter.

Relative Messungen der oben genannten Parameter bieten den Vorteil, dass der Einfluss unterschiedlicher Blutflüsse sowie Rückflusswiderstände entfällt.

Die Analyse des Frequenzspektrums einzelner Druckmesswerte hat weiterhin gezeigt, dass eine Änderung der Permeabilität sich auf die Amplituden der harmonischen Frequenzen auswirkt. Gleiches gilt für Flussänderungen in Blutflussrichtung.

In einer bevorzugten Ausführungsform wird das Frequenzspektrum einzelner Signale sowie die relative Änderung in Bezug auf ein zweites Signal ermittelt. Durch Auswertung der beiden Frequenzspektren kann eine Aussage über die Flusseigenschaften in Blutflussrichtung, Dialysatflussrichtung oder in Transmembranrichtung getroffen werden, wobei Blutflussrichtung und Transmembranrichtung bevorzugt sind.

Bevorzugt ist dabei die Messung von mindestens zwei Druckmesswerten in der Blutbehandlungseinheit an mindestens zwei Drucksensoren, also mindestens ein Druckmesswert pro Drucksensor.

Des weiteren betrifft die vorliegende Offenbarung ein Verfahren zur bestmöglichen Erreichung eines vorbestimmten Substitutionsziels bei einer Blutbehandlungseinheit, das die folgenden Schritte umfasst:

a) Bestimmung der Filtereigenschaften durch Ermittlung von mindestens zwei Druckmesswerten an mindestens zwei Drucksensoren innerhalb der Blutbehandlungseinheit mit einer Referenzlösung;

b) optional Vergleich der gemessenen Druckmesswerte mit Referenzwerten desselben Patienten, welche in früheren Dialysesitzungen erhoben wurden oder mit Referenzwerten einer Patientengruppe,

c) Berechnung des Substitutionsziels auf Grundlage der Werte gemäß Schritt a) und optional Schritt b),

d) Messen von Druckmesswerten an mindestens zwei Drucksensoren während einer Blutbehandlung,

e) Vergleich der gemäß Schritt d) gemessenen Druckmesswerte mit Referenzdruckmesswerten, und

f) Anpassung der Flussrate von Blut, Dialysat und/oder Substituat bei Abweichung von mindestens einem gemessenen Druckmesswert von dem Referenzdruckmesswert, um bei festgelegter Behandlungszeit das Substitutionsziel bestmöglichst zu erreichen oder bei festgelegtem Substitutionsziel die Behandlungsdauer möglichst wenig zu verändern.

Eine weitere Ausführungsform der vorliegenden Offenbarung betrifft ein Verfahren zur bestmöglichen Erreichung eines berechneten Substitutionsziels bei einer Blutbehandlungseinheit mit einem Dialysator, mindestens zwei Drucksensoren, einer zentralen Recheneinheit und einer Speichereinheit, wobei das Verfahren die folgenden Schritte umfasst:

a) Bestimmung der Filtereigenschaften durch Ermittlung von mindestens zwei Druckmesswerten an den mindestens zwei Drucksensoren innerhalb der Blutbehandlungseinheit mit einer Referenzlösung;

b) optional Vergleich der gemessenen Druckmesswerte mit auf der Speichereinheit hinterlegten Referenzwerten desselben Patienten, welche in früheren Dialysesitzungen erhoben wurden oder mit auf der Speichereinheit hinterlegten Referenzwerten einer Patientengruppe,

c) Berechnung des Substitutionsziels auf Grundlage der Werte gemäß Schritt a) und optional Schritt b),

d) Messen von Druckmesswerten an den mindestens zwei Drucksensoren während einer Blutbehandlung,

e) Vergleich der gemäß Schritt d) gemessenen Druckmesswerte mit auf der Speichereinheit hinterlegten Referenzdruckmesswerten, und

f) Anpassung der Flussrate von Blut, Dialysat und/oder Substituat bei Abweichung von mindestens einem gemessenen Druckmesswert von dem auf der Speichereinheit hinterlegten Referenzdruckmesswert, um bei festgelegter Behandlungszeit das Substitutionsziel bestmöglichst zu erreichen oder bei festgelegtem Substitutionsziel die Behandlungsdauer möglichst wenig zu verändern.

Als Referenzdruckmesswerte dienen vorzugsweise solche, die während einer Blutbehandlung oder während Blutbehandlungen erhoben wurden, in der oder in denen das Substitutionsziel erreicht worden ist.

Bei dem Vergleich der gemessenen Druckmesswerte mit den auf der Speichereinheit hinterlegten Referenzdruckmesswerten ist selbstverständlich, dass Druckmesswerte gemessen an demselben Drucksensor und zu ungefähr derselben Zeit während der Blutbehandlung miteinander verglichen werden.

Dabei sind in allen Ausführungsformen der erfindungsgemäßen Blutbehandlungseinheit folgende Formulierungen von Schritt f) auch möglich:

f) Anpassung der Flussrate(n) von Blut oder Dialysat oder Substituat oder von Blut und Dialysat oder Dialysat und Substituat oder Blut und Substituat oder von Blut und Dialysat und Substituat bei Abweichung von mindestens einem gemessenen Druckmesswert von dem entsprechenden Referenzdruckmesswert, um bei festgelegter Behandlungszeit das Substitutionsziel bestmöglich zu erreichen oder bei festgelegtem Substitutionsziel die Behandlungsdauer zu verändern. oder

f) Anpassung der Flussrate von Blut oder Dialysat oder Substituat oder der Flussraten von Blut und Dialysat oder Dialysat und Substituat oder Blut und Substituat oder Blut und Dialysat und Substituat bei Abweichung von mindestens einem gemessenen Druckmesswert von dem entsprechenden auf der Speichereinheit hinterlegten Referenzdruckmesswert, um bei festgelegter Behandlungszeit das Substitutionsziel bestmöglich zu erreichen oder bei festgelegtem Substitutionsziel die Behandlungsdauer zu verändern.

In allen Ausführungsformen des Verfahrens können die hinterlegten Referenzdruckmesswerte gemäß Schritt e) bei Blutbehandlungen erhoben worden sein, in denen das Substitutionsziel während der Behandlungszeit erreicht worden ist.

In einer weiteren bevorzugten Ausführungsform des Verfahrens sind die Daten, mit denen verglichen wird, auf einer Speichereinheit hinterlegt, und es werden die notwendigen Anpassungen und entsprechende Steuersignale über eine zentrale Recheneinheit ausgegeben. Das Verfahren zur bestmöglichen Erreichung eines vorbestimmten Substitutionsziels bei einer Blutbehandlungseinheit mit einem Dialysator, mindestens zwei Drucksensoren, einer zentrale Recheneinheit und einer Speichereinheit, umfasst dann die folgenden Schritte:

a) Bestimmung der Filtereigenschaften durch Ermittlung von mindestens zwei Druckmesswerten an den mindestens zwei Drucksensoren innerhalb der Blutbehandlungseinheit mit einer Referenzlösung;
b) optional Vergleich der gemessenen Druckmesswerte mit auf der Speichereinheit hinterlegten Referenzwerten desselben Patienten, welche in früheren Dialysesitzungen erhoben wurden oder mit auf der Speichereinheit hinterlegten Referenzwerten einer Patientengruppe,
c) Berechnung des Substitutionsziels auf Grundlage der Werte gemäß Schritt a) und optional Schritt b),
d) Messen von Druckmesswerten an den mindestens zwei Drucksensoren während einer Blutbehandlung,
e) Vergleich der gemäß Schritt d) gemessenen Druckmesswerte mit auf der Speichereinheit hinterlegten Referenzdruckmesswerten, und
f) Ausgabe eines Signals zum Hinweise auf die Notwendigkeit der Anpassung oder automatische Anpassung der Flussrate von Blut, Dialysat und/oder Substituat bei Abweichung von mindestens einem gemessenen Druckmesswert von dem entsprechenden auf der Speichereinheit hinterlegten Referenzdruckmesswert, um bei festgelegter Behandlungszeit das Substitutionsziel bestmöglichst zu erreichen oder bei festgelegtem Substitutionsziel die Behandlungsdauer möglichst wenig zu verändern.

Das Messen der Druckwerte kann in allen Ausführungsformen der erfindungsgemäßen Vorrichtung zeitgleich oder zeitversetzt erfolgen.

In dem Verfahren ist es bevorzugt, wenn in Schritt c) mindestens zwei zeitgleiche Druckmesswerte, die an mindestens zwei der Drucksensoren ([PB1], [PB2], [PD1] und [PD2]) gemessen werden, für die Berechnung des Substitutionsziels verwendet werden.

In dem Verfahren ist es weiterhin bevorzugt, wenn in Schritt c) mindestens zwei zeitversetzte Druckmesswerte, die an mindestens einem der Druckmesssensoren ([PB1], [PB2], [PD1] und [PD2]) gemessen werden, für die Berechnung des Substitutionsziels verwendet werden.

In dem Verfahren ist es weiterhin bevorzugt, wenn in Schritt c) der Blutfluss, der Hämatokrit des Patienten, die Gesamtproteinkonzentration des Patienten oder die Patientenhistorie oder eine Kombination davon in die Bestimmung des optimalen Substitutionsziels mit einbezogen werden.

In dem Verfahren ist es weiterhin bevorzugt, wenn in Schritt c) die Patientenhistorie dahingehend einbezogen wird, dass für den Fall, dass mit einem für mindestens eine vorhergehende Dialysesitzung als optimal bestimmten Substitutionsziel bei einem Patienten Komplikationen aufgetreten sind, das für die anstehende Dialysesitzung ermittelte optimale Substitutionsziel unter diesen als kritisch erkannten Wert herabgesetzt wird, falls es gleich oder größer als dieser kritische Wert ist.

Ferner ist bevorzugt, wenn die Dauer der Blutbehandlung, d.h. die Dauer der Dialysesitzung festgelegt wird und das berechnete Substitutionsziel innerhalb der festgelegten Dauer bestmöglichst erreicht wird. Dies gewährleistet einen reibungslosen Ablauf in den Dialysestationen und ein bestmögliches Resultat für den Patienten.

Weiter bevorzugt ist die Messung von Druckmesswerten an drei Drucksensoren in einer Blutbehandlungseinheit mit einem Dialysator, mindestens drei Drucksensoren, einer zentralen Recheneinheit und einer Speichereinheit.

Besonders bevorzugt ist die Messung von Druckmesswerten an vier Drucksensoren in einer Blutbehandlungseinheit mit einem Dialysator, mindestens vier Drucksensoren, einer zentralen Recheneinheit und einer Speichereinheit. Somit betrifft die vorliegende Erfindung auch eine Blutbehandlungseinheit mit einem Dialysator, mindestens vier Drucksensoren, einer zentralen Recheneinheit und einer Speichereinheit, geeignet um folgendes Verfahren auszuführen:

a) Bestimmung der Filtereigenschaften durch Ermittlung von mindestens vier Druckmesswerten an mindestens vier Drucksensoren innerhalb der Blutbehandlungseinheit mit einer Referenzlösung,
b) optional Vergleich der gemessenen Druckmesswerte mit entsprechenden auf der Speichereinheit hinterlegten Referenzwerten desselben Patienten, welche in früheren Dialysesitzungen erhoben wurden oder mit auf der Speichereinheit hinterlegten Referenzwerten einer Patientengruppe,
c) Berechnung eines Substitutionsziels auf Grundlage der Werte gemäß Schritt a) und optional Schritt b),
d) Messen von Druckmesswerten an den mindestens vier Drucksensoren während einer Blutbehandlung,
e) Vergleich der gemäß Schritt d) gemessenen Druckmesswerte mit entsprechenden auf der Speichereinheit hinterlegten Referenzdruckmesswerten, und
f) Anpassung der Flussrate von Blut, Dialysat und/oder Substituat bei Abweichung von mindestens einem gemessenen Druckmesswert von dem entsprechenden auf der Speichereinheit hinterlegten Referenzdruckmesswert, um bei festgelegter Behandlungszeit das Substitutionsziel bestmöglichst zu erreichen oder bei festgelegtem Substitutionsziel die Behandlungsdauer möglichst wenig zu verändern.

Wie hierin verwendet, bedeutet der Ausdruck "von vier Druckmesswerten an vier Drucksensoren", dass ein Druckmesswert pro Drucksensor gemessen wird, also insgesamt 4 Druckmesswerte erhalten werden und nicht, dass an 4 Drucksensoren jeweils 4 Druckmesswerte gemessen werden, also insgesamt 16 Druckmesswerte erhalten werden. Gleiches gilt für den Begriff "von (mindestens) drei Druckmesswerten an (mindestens) drei Drucksensoren", wo insgesamt also (mindestens) drei Druckmesswerte erhalten werden (nämlich einer pro Drucksensor) und auch für den Begriff "von (mindestens) zwei Druckmesswerten an (mindestens) zwei Drucksensoren", wo insgesamt also (mindestens) zwei Druckmesswerte erhalten werden.

Wenn die Blutbehandlungseinheit vier Drucksensoren aufweist, so befindet sich einer dialysatseitig am Einlaß des Dialysators [PD1] und der zweite dialysatseitig am Auslaß des Dialysators [PD2] und der dritte blutseitig am Einlaß des Dialysators [PB1] und der vierte blutseitig am Auslaß des Dialysators [PB2].

Weist die Blutbehandlungseinheit drei erfindungsgemäß verwendete Drucksensoren auf, so fehlt einer der vier vorgenannten Drucksensoren [PD1], [PD2], [PB1] und [PB2]. Weist die Blutbehandlungseinheit zwei erfindungsgemäß verwendete Drucksensoren auf, so fehlen zwei der vier vorgenannten Drucksensoren [PD1], [PD2], [PB1] und [PB2].

In einer weiteren Ausführungsform des Verfahrens werden die Drucksignale einer Blutpumpe zur erfindungsgemäßen Differenzierung von Systemänderungen verwendet.

In einer weiteren Ausführungsform werden die Drucksignale einer Bilanzkammer BK zur erfindungsgemäßen Differenzierung von Systemänderungen ermittelt.

Eine bevorzugte Ausführungsform des Verfahrens umfasst weiterhin den Schritt die Druckmesswerte von äußeren Einflussparametern zu bereinigen. Der Begriff "*äußere Einflussparameter*" wie hierin verwendet umfasst Flussgeschwindigkeiten, Höhenänderung des Patientenzugangs oder erhöhter Widerstand im Patientenrückfluss. Mit Höhenänderung ist gemeint, dass der Patient im Vergleich zum Tangentialflussfilters TFF seine räumliche Lage ändert. Eine weitere wichtige Einflussgröße ist der Blutdruck des Patienten. So tritt beispielsweise relativ häufig Hypotension auf. Zur Kompensation derartiger Änderungen können die relativen Werte betrachtet werden. Zeigen sich in den einzelnen Signalen Änderungen, die auf eine Änderung äußerer Einflüsse schließen lassen, wird dieses Signal für den Zeitraum der Änderung als "kritisch" gekennzeichnet und im Zweifelsfall nicht zur Auswertung herangezogen. Auf dieselbe Weise wird vorgegangen, wenn blut- und dialysatseitige Signale in Relation zueinander gesetzt werden.

Eine Ausführungsform zur Ermittlung von Druckmesswerten zur erfindungsgemäßen Differenzierung von Systemänderungen umfasst folgende Schritte:

(a) mindestens zwei Druckmesswerte werden in einer Blutbehandlungseinheit zeitgleich oder zeitlich versetzt ermittelt,

(b) Korrektur der Druckmesswerte von äußeren Einflussparametern,

(c) Auswertung der mindestens zwei korrigierten Druckmesswerte aus (b), zum Treffen einer Aussage über die Flusseigenschaften in Blutflussrichtung oder in Transmembranrichtung.

Es können nicht nur die Signale zweier unterschiedlicher Druckaufnehmer zueinander in Beziehung gesetzt werden, sondern auch der zeitliche Verlauf eines Signals. Als Beispiel sei die Überwachung der Amplitude auf der Dialysatseite genannt. Wird die Amplitude kleiner, hat sich entweder die Permeabilität der Membran oder die effektive Membranoberfläche verringert, oder das Eingangssignal ist schwächer geworden. Hierbei kann aber keine Offsetkorrektur Berücksichtigung finden. Äußere Einflüsse würden sich direkt in den Messergebnis niederschlagen können. Durch eine relative Betrachtung im Vergleich zur Amplitude des Eingangssignals und bei gleichzeitiger Überwachung des blutseitigen Ausgangs lässt sich das Problem jedoch recht genau identifizieren.

Bei allen hierin beschriebenen Verfahren ist es bevorzugt mehr als zwei Druckmesswerte und insbesondere 4 Druckmesswerte zeitgleich oder zeitversetzt an den Ein- und Auslässen des Tangentialflussfilters TFF zu messen, so wie durch die Drucksensoren [PB1], [PB2], [PD1] und [PD2] vorgenommen.

Die Offenbarung umfasst weiterhin auch eine Vorrichtung zum Messen von Druckmesswerten in einer Vorrichtung zur Behandlung von Blut, welche die Dialyseeffizienz und Wirtschaftlichkeit der Dialyse steigert, durch Differenzierung zwischen Systemänderungen, die in Blutflussrichtung oder in Transmembranrichtung auftreten, umfassend mindestens einen Drucksensor zur Messung von Druckmesswerten.

Sämtliche zuvor beschriebene Ausführungsformen und Vorteile beziehen sich vorteilhaft auch auf das Verfahren und die Vorrichtung zur Messung von Druckmesswerten in einer Blutbehandlungseinheit, welche die Dialyseeffizienz und Wirtschaftlichkeit der Dialyse steigert und zugleich sicherstellt, dass dem Patienten das der jeweilige Leistungsfähigkeit des Dialysegerätes entsprechende Volumen an Substituatlösung zugeführt wird. Dies dient auch der Sicherheit des Patienten und seinem körperlichen Wohlsein während und nach der jeweiligen Dialysesitzung.

Die Zusammensetzung der Druckmesswerte ist für das Verfahren nicht erheblich. Erfindungsgemäß können Druckmesswerte aus einzelnen Quellen genutzt werden, aber auch Druckmesswerte, die sich aus einer Vielzahl von Quellen zusammensetzen.

In einigen Ausführungsformen kann es vorteilhaft sein, wenn aus der Summe der Druckmesswerte nur einer ermittelt wird bzw. aus der Summe der Druckmesswerte nur einer herausgefiltert wird, um einen gültigen Wert für die übrigen Druckmesswerte zu ermitteln. Dies kann z.B. der Fall sein, wenn ein sehr unregelmäßiger Druckmesswert die Messung der anderen überlagern würde oder wenn ein bestimmtes Drucksignal sich aufgrund seiner Eigenschaften besonders für die Messung eignet. Solche Einrichtungen zur Korrektur der Druckmesswerte sind aus dem Stand der Technik hinreichend bekannt.

Zusammengefasst läst sich das Verfahren zur Festlegung und bestmöglichen Realisierung Substitutionsziels bei einen Dialysator, mindestens zwei Drucksensoren, eine zentrale Recheneinheit und eine Speichereinheit umfassenden Blutbehandlungseinheiten anhand der folgenden Schritte darstellen:

a) Bestimmen der Filtereigenschaften oder des Filtertyps durch Ermittlung von Druckmesswerten innerhalb der Blutbehandlungseinheit mit einer Referenzlösung;

b) Vergleich der gemessenen Druckmesswerte mit auf einer Speichereinheit hinterlegten entsprechenden Messwerten

c) Festlegung des Substitutionsziels auf Grundlage der Informationen gemäß a) und optional b);

d) Messen von Druckmesswerten in einer Blutbehandlungseinheit während einer Blutbehandlung,

e) Vergleich der gemessenen Druckmesswerte mit entsprechenden auf der Speichereinheit hinterlegten Referenzdruckmesswerten; und

f) Bei Abweichung von mindestens einem gemessenen Druckmesswert von dem Referenzdruckmesswert erfolgt Anpassung der Flussrate(n) und/oder des Drucks / der Drücke von Blut, Dialysat und/oder Substituat, um bei festgelegter Behandlungszeit das Substitutionsziel bestmöglich zu erreichen oder bei festgelegtem Substitutionsziel die Behandlungsdauer möglichst wenig zu verändern,

wobei die zentrale Recheneinheit konfiguriert ist, auf Grundlage der über die Referenzlösung gemessenen Druckmesswerte spezifische Filtereigenschaften zu erkennen und entsprechend der erkannten Filtereigenschaften vor Beginn einer Behandlung ein bestmögliches Substitutionsziel zu bestimmen und während der Blutbehandlung Druckmesswerte zu erfassen und mit Referenzdruckmesswerten zu vergleichen und bei Abweichungen Steuersignale zu generieren, um die Anpassung der Flussrate(n) und/oder des Drucks / der Drücke von Blut, Dialysat und/oder Substituat vorzuschlagen oder vorzunehmen, um das Substitutionsziel bei festgelegter Behandlungszeit bestmöglichst zu erreichen oder bei festgelegtem Substitutionsziel die Behandlungsdauer möglichst wenig zu verändern.

Die vorliegende Offenbarung umfasst weiterhin eine Vorrichtung zum Messen von Druckmesswerten in einer Blutbehandlungseinheit, um Systemänderungen in Filtrationsprozessen zu messen und zwischen Systemänderungen zu differenzieren, die in Blutflussrichtung oder in Transmembran-Richtung auftreten, umfasst mindestens zwei Drucksensoren zur Messung von Druckmesswerten.

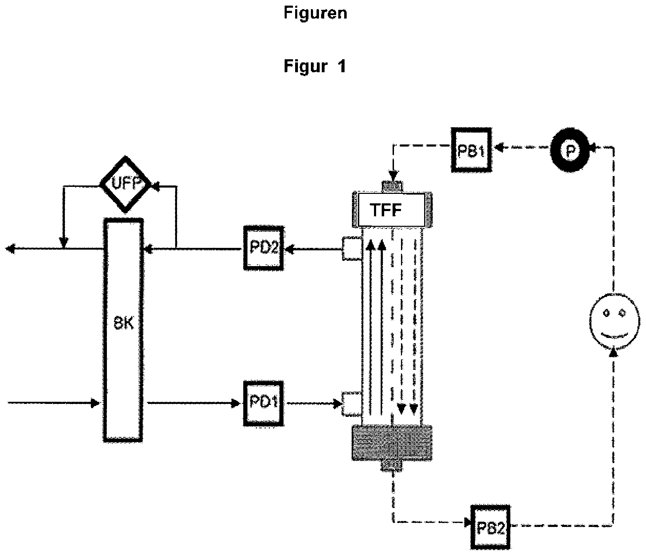

Bei der erfindungsgemäßen Blutbehandlungseinheit handelt es sich vorzugsweise um eine Vorrichtung zur extrakorporalen Blutbehandlung mit einem Dialysator (Tangentialflussfilter, TFF), der durch seine semipermeable Membran (11) in eine erste und zweite Kammer geteilt ist, wobei die erste Kammer (12) in einem Dialysierflüssigkeitsweg angeordnet ist und die zweite Kammer (13) mittels einer Blutzuführleitung (14) und einer Blutabführleitung (15) mit dem Blutkreislauf eines Patienten (☺) verbindbar ist, einem Zulauf (20) für frische Dialysierflüssigkeit, einem Ablauf (30) für verbrauchte Dialysierflüssigkeit, einer Pumpe in der Blutzufuhrleitung zwischen Patientenzugang (☺) und TFF, sowie einer Bilanzkammer (BK) und einer Ultrafiltrationspumpe (UFP) im Dialysierflüssigkeitsweg (Figur 1).

In der Blutbehandlungseinheit können mindestens eine, bevorzugt mindestens zwei, ebenfalls bevorzugter mindestens drei und am meisten bevorzugt mindestens vier Drucksensoren angebracht sein. Dabei ist die Definition und Anordnung der Drucksensoren wie folgt: [PB1] ist der Drucksensor auf der blutseitigen Eingangsseite des TFF bzw. in der Blutzufuhrleitung (14), [PB2] der Drucksensor auf der blutseitigen Ausgangsseite des TFF bzw. in der Blutabführleitung [15], [PD1] der Drucksensor auf der Eingangsseite der Dialyseflüssigkeit des TFF bzw. dem Zulauf für frische Dialysierflüssigkeit (20) und [PD2] der Druck auf der Ausgangseite der Dialyseflüssigkeit des TFF bzw. dem Ablauf für verbrauchte Dialysierflüssigkeit (30).

In einer bevorzugten Ausführungsform sind blutseitig ein Drucksensor [PB1] zwischen einer Pumpe P und einem Filter TFF und ein weiterer Drucksensor [PB2] zwischen dem Filter TFF und dem Patienten ☺ angebracht und dialysatseitig ein Drucksensor [PD2] hinter dem Ablauf des Filters TFF und ein weiterer Drucksensor [PD1] vor dem Zulauf zum Filter TFF angebracht.

In einer weiteren Ausführungsform umfasst die erfindungsgemäße Vorrichtung weiterhin eine Vorrichtung zum Auswerten der Messdaten. Dies kann bevorzugt eine Speichereinheit und/oder eine zentrale Recheneinheit sein, z.B. eine CPU, welche Änderungen der gemessenen Druckmesswerte zu vorgegebenen Referenzwerten und/oder Änderungen zu vorher gemessenen Ausgangswerten berechnet.

In weiteren Ausführungsformen umfasst die erfindungsgemäße Vorrichtung lediglich einen Drucksensor. Eine Aussage über eine Systemänderung im Filtrationsprozess wird anhand der zeitlichen Entwicklung der Messwerte dieses einen Drucksensors getätigt. Dieser Drucksensor kann [PB1], [PB2], [PD1] oder [PD2] sein. Erfindungsgemäß kann aber die zeitliche Entwicklung der Messwerte an bis zu vier Drucksensoren [PB1], [PB2], [PD1] und [PD2] verfolgt und zeitlich miteinander in Verbindung gesetzt werden.

In Tabelle 2 sind beispielhaft einige Musterkonstellationen für auftretende Druckveränderungen im Betrieb einer Dialysemaschine und die ihnen zugrundeliegenden Störungen zusammengestellt:

**Tabelle 2**

| PB1 | PB2 | PD1 | PD2 | Störung |
| --- | --- | --- | --- | --- |
| - | 0 | 0 | 0 | Blocking wird reduziert |
| - | 0 | 0 | 0 | Verringerung der UF-Rate verringert die Hämokonzentration |
| -- | -- | -- | -- | akute Flussverengung vor PD1 |
| -- | -- | 0 | -- | akute Flussverengung zwischen PD1 und Filter |
| + | 0 | 0 | 0 | geringere Durchflussfläche für das Blut verursacht durch Clotting |
| + | 0 | 0 | 0 | Erhöhung der UF-Rate hat eine stärkere Hämokonzentration zur Folge |
| + | 0 | - | - | Sekundärmembranbildung |
| + | + | + | + | Druckniveau auf Patientenseite steigt (z.B. höherer Eingangshämatokrit, Änderung der Armposition) |
| + | + | + | + | Flussveränderung im Zugang |
| ++ | 0 | 0 | 0 | Probleme am Schlauchsystem zwischen PB1 und Filter |
| ++ | 0 | ++ | ++ | Probleme am Schlauchsystem zwischen Filter und PB2 |
| ++ | ++ | ++ | ++ | Probleme am Schlauchsystem hinter PB2 oder Flussveränderung im Zugang |
| 0 | 0 | 0 | -- | akute Flussverengung zwischen Filter und PD2 |

+ steigt an
++ steigt stark (schnell) an
- fällt ab
-- fällt stark (schnell) ab
0 bleibt unverändert

Durch Clotting kann sich beispielsweise die effektive Membranoberfläche verringern. Infolgedessen fallen die dialysatseitigen Drücke, um die UF-Rate aufrechtzuerhalten. Dementsprechend kann ein Anstieg von PB1 zur Folge haben, daß PD1 und PD2 gleichermaßen fallen, da die Bilanzierung aufrechterhalten werden muß und somit der gleiche Fluss durch eine verringerte Fläche fließt.

Eine bevorzugte Ausführungsform umfasst die Vorrichtung zur Behandlung von Blut einen Tangentialflussfilter TFF, eine Pumpe P, und mindestens zwei Drucksensoren ([PB1], [PB2] oder [PD1], [PD2] oder [PB1], [PD1] oder [PB1], [PD2] oder [PB2], [PD1]oder [PB2], [PD2]), wobei die Drucksensoren ([PB1], [PB2] oder [PD1], [PD2] oder [PB1], [PD1] oder [PB1], [PD2] oder [PB2], [PD1]oder [PB2], [PD2]) dem Tangentialflussfilter TFF unmittelbar vorgeschaltet und/oder unmittelbar nachgeschaltet sind.

In einer weiter bevorzugten Ausführungsform umfasst die Vorrichtung zur Behandlung von Blut einen Tangentialflussfilter TFF, eine Pumpe P, und mindestens drei Drucksensoren ([PB1], [PB2], [PD1] oder [PB1], [PB2], [PD2] oder [PD1], [PD2], [PB1] oder [PD1], [PD2], [PB2]), wobei die Drucksensoren ([PB1], [PB2], [PD1] oder [PB1], [PB2], [PD2] oder [PD1], [PD2], [PB1] oder [PD1], [PD2], [PB2]) dem Tangentialflussfilter TFF unmittelbar vorgeschaltet und/oder unmittelbar nachgeschaltet sind.

Noch bevorzugter ist eine Vorrichtung zur Behandlung von Blut umfassend einen Tangentialflussfilter TFF, eine Pumpe P, und vier Drucksensoren [PB1], [PB2], [PD1] und [PD2], wobei die Drucksensoren [PB1], [PD1] dem Tangentialflussfilter TFF unmittelbar vorgeschaltet und die Drucksensoren [PB2], [PD2] dem Tangentialflussfilter TFF unmittelbar nachgeschaltet sind.

Die vorliegende Erfindung bezieht sich bevorzugt auf eine Blutbehandlungseinheit mit einem Dialysator und mindestens zwei Drucksensoren, geeignet um folgendes Verfahren auszuführen:

a) Messung von mindestens zwei Druckmesswerten an mindestens zwei Drucksensoren innerhalb der Blutbehandlungseinheit mit einer Referenzlösung,

b) Bestimmung der Filtereigenschaften oder des Filtertyps durch Vergleich der in Schritt a) gemessenen Druckmesswerte mit hinterlegten entsprechenden Druckmesswerten einer Filterart oder eines Filtertyps,

c) Berechnung eines Substitutionsziels auf Grundlage der Informationen gemäß a) und b),

wobei auf der Grundlage der mittels Referenzlösung ermittelten Druckmesswerte spezifische Filtereigenschaften erkannt und entsprechend der erkannten Filtereigenschaften vor Beginn einer Blutbehandlung ein optimales Substitutionsziel bestimmt werden kann.

Die Vorrichtung ist also geeignet, bei Inbetriebnahme des Blutbehandlungseinheit durch Aufnahme von Druckmesswerten die Eigenschaften des verwendeten Filters zu ermitteln, oder sogar den Filtertyp zu erkennen sofern typspezifische Daten hinterlegt sind, und dementsprechend eine Voraussage über das optimale Substitutionsziel zu treffen, also die Menge an unter bestmöglichen Bedingungen benötigten Menge an Substituatflüssigkeit zu ermitteln. Dabei dienen die hinterlegten Messwerte dem Abgleich mit den aktuellen Messwerten und dienen damit der Bestimmung des Substitutionsziels bzw. zu Aussagen über den Zustand des Systems und/oder des Filters.

Die vorliegende Erfindung bezieht sich weiterhin bevorzugt auf eine Blutbehandlungseinheit mit einem Dialysator und mindestens zwei Drucksensoren, geeignet um folgendes Verfahren auszuführen:

a) Bestimmung der Filtereigenschaften durch Ermittlung von mindestens zwei Druckmesswerten an mindestens zwei Drucksensoren innerhalb der Blutbehandlungseinheit mit einer Referenzlösung,

b) optional Vergleich der gemessenen Druckmesswerte mit auf der Speichereinheit hinterlegten Referenzwerten desselben Patienten, welche in früheren Dialysesitzungen erhoben wurden oder mit auf der Speichereinheit hinterlegten Referenzwerten einer Patientengruppe,

c) Festlegung eines Substitutionsziels auf Grundlage der gemessenen Werte gemäß Schritt a) und optional Schritt b),

d) Aufnahme von mindestens zwei Druckmesswerten an mindestens zwei Drucksensoren während einer Blutbehandlung,

e) Vergleich der Druckmesswerte mit hinterlegten Referenzdruckmesswerten; und

f) Anpassung der Flussrate(n) von Blut, Dialysat und/oder Substituat bei Abweichung von mindestens einem gemessenen Druckmesswert von dem Referenzdruckmesswert, um bei festgelegter Behandlungszeit das in Schritt c) festgelegte Substitutionsziel bestmöglich zu erreichen oder bei Festhalten am festgelegten Substitutionsziel die Behandlungsdauer zu verändern,

wobei auf der Grundlage der mittels Referenzlösung gemessenen Druckmesswerte spezifische Filtereigenschaften erkannt und entsprechend der erkannten Filtereigenschaften vor Beginn einer Behandlung ein bestmögliches Substitutionsziel bestimmt werden kann. Das System ist weiterhin so konfiguriert, dass es während einer Blutbehandlung Druckmesswerte erfassen, mit Referenzdruckmesswerten vergleichen und bei Abweichungen Steuersignale generieren kann, um das Substitutionsziel bestmöglich zu erreichen.

In allen Ausführungsformen der erfindungsgemäßen Blutbehandlungseinheit können die hinterlegten Referenzdruckmesswerte gemäß Schritt e) bei Blutbehandlungen erhoben worden sein, in denen das Substitutionsziel während der Behandlungszeit erreicht worden ist.

Es ist im Sinne der Erfindung weiterhin besonders bevorzugt, wenn eine Speichereinheit und eine zentrale Recheneinheit Teile der Blutbehandlungseinheit sind. Die vorliegende Erfindung bezieht sich darum besonders bevorzugt auf eine Blutbehandlungseinheit mit einem Dialysator, mindestens zwei Drucksensoren, mit mindestens einer Speichereinheit und mindestens einer zentralen Recheneinheit, geeignet um folgendes Verfahren auszuführen:

a) Bestimmung der Filtereigenschaften durch Ermittlung von mindestens zwei Druckmesswerten an mindestens zwei Drucksensoren innerhalb der Blutbehandlungseinheit mit einer Referenzlösung,

b) optional Vergleich der gemessenen Druckmesswerte mit auf der Speichereinheit hinterlegten Referenzwerten desselben Patienten, welche in früheren Dialysesitzungen erhoben wurden oder mit auf der Speichereinheit hinterlegten Referenzwerten einer Patientengruppe,

c) Berechnung eines Substitutionsziels auf Grundlage der Werte gemäß Schritt a) und optional Schritt b),

wobei die Speichereinheit konfiguriert ist, auf Grundlage der über die Referenzlösung gemessenen Druckmesswerte spezifische Filtereigenschaften zu erkennen und entsprechend der erkannten Filtereigenschaften vor Beginn einer Behandlung ein bestmögliches Substitutionsziel zu bestimmen.

Die Erfindung bezieht sich weiterhin besonders bevorzugt auf eine Blutbehandlungseinheit mit einem Dialysator, mindestens zwei Drucksensoren, mit mindestens einer Speichereinheit und mindestens einer zentralen Recheneinheit, geeignet um folgendes Verfahren auszuführen:

a) Bestimmung der Filtereigenschaften durch Ermittlung von mindestens zwei Druckmesswerten an mindestens zwei Drucksensoren innerhalb der Blutbehandlungseinheit mit einer Referenzlösung,

b) optional Vergleich der gemessenen Druckmesswerte mit auf der Speichereinheit hinterlegten Referenzwerten desselben Patienten, welche in früheren Dialysesitzungen erhoben wurden oder mit auf der Speichereinheit hinterlegten Referenzwerten einer Patientengruppe,

c) Berechnung eines Substitutionsziels auf Grundlage der Werte gemäß Schritt a) und optional Schritt b),

d) Messen von Druckmesswerten an mindestens zwei Drucksensoren während einer Blutbehandlung,

e) Vergleich der gemäß Schritt d) gemessenen Druckmesswerte mit auf der Speichereinheit hinterlegten entsprechenden Referenzdruckmesswerten, und

f) Anpassung der Flussrate von Blut, Dialysat und/oder Substituat bei Abweichung von mindestens einem gemessenen Druckmesswert von dem auf der Speichereinheit hinterlegten entsprechenden Referenzdruckmesswert, um bei festgelegter Behandlungszeit das Substitutionsziel bestmöglichst zu erreichen oder bei festgelegtem Substitutionsziel die Behandlungsdauer möglichst wenig zu verändern,

wobei die Speichereinheit konfiguriert ist, auf Grundlage der über die Referenzlösung gemessenen Druckmesswerte spezifische Filtereigenschaften zu erkennen und entsprechend der erkannten Filtereigenschaften vor Beginn einer Behandlung ein optimales Substitutionsziel zu berechnen, und die zentrale Recheneinheit weiterhin so konfiguriert ist, dass sie während einer Blutbehandlung Druckmesswerte erfassen, mit entsprechenden Referenzdruckmesswerten vergleichen und bei Abweichungen Steuersignale generieren kann, um das Substitutionsziel bestmöglich zu erreichen.

Die Speichereinheit und die zentrale Recheneinheit können dabei jeweils eine zentrale Recheneinheit sein, eine Festplatte, oder eine elektronische Steuereinheit sein. Die Speichereinheit dient besonders und bevorzugt zur Identifikation bestimmter Filtermerkmale, die zur Bestimmung des Substitutionsziels geeignet sind. Dafür wird auf Informationen zugegriffen, die beispielsweise in Form einer Tabelle oder einer Matrix hinterlegt sind, und bevorzugt durch eine Zuordnungsfunktion oder ein Steuerprogamm zur Bestimmung des Substitutionsziels dienen. Die Informationen auf der Speichereinheit dienen ganz besonders bevorzugt zur Identifizierung und Zuordnung bestimmter Filtereigenschaften, so dass auf Grund der gemessenen Werte die Eigenschaften des Filters erfasst und der Filtertyp identifiziert werden kann.

Dabei ist es besonders bevorzugt, wenn der Filtertyp innerhalb der Blutbehandlungseinheit in einem Testlauf mit einer Referenzlösung auf der Basis der ermittelten Druckmesswerte erkannt wird.

Hierfür werden jeweils mindestens zwei zeitgleiche Druckmesswerte, die an mindestens zwei der Druckmesssensoren [PB1], [PB2], [PD1] und [PD2] gemessen werden, für die Berechnung des Substitutionsziels verwendet.

In weiteren Ausführungsformen werden jeweils mindestens zwei zeitversetzte Druckmesswerte, die an mindestens einem der Druckmesssensoren [PB1], [PB2], [PD1] und [PD2] gemessen werden, für die Berechnung des Substitutionsziels verwendet.

Alternativ dazu kann mindestens ein gemessener Druckmesswert, der an mindestens einem der Druckmesssensoren [PB1], [PB2], [PD1] und [PD2] gemessen wird, mit jeweils einem dem Druckmesssensor entsprechenden, auf der Speichereinheit hinterlegten Druckmesswert abgeglichen werden, um für die Berechnung des Substitutionsziels verwendet zu werden.

Die auf der Speichereinheit hinterlegte Information kann bevorzugt eine Matrix, eine Tabelle oder eine Zuordnungsfunktion sein. Bei einer Matrix oder einer Tabelle wird ein bestimmter Druckmesswert beispielsweise einer bestimmten Flussrate oder einem bestimmten Volumen oder einem bestimmten Strömungsverhalten zugeordnet. Die Zuordnungsfunktion sucht auf der Basis der ermittelten Werte entsprechende hinterlegte Werte mittels eines Suchalgorithmus.

Die Speichereinheit kann gemäß der Erfindung weiterhin Informationen bezüglich zusätzlicher Patientendaten enthalten. Diese Daten können sich beispielsweise auf den Blutdruck des Patienten, den Blutfluss, der Hämatokrit des Patienten, die Gesamtproteinkonzentration des Patienten oder die Patientenhistorie beziehen, die allein oder in Kombination zur Bestimmung des bestmöglichen Substitutionsziels mit herangezogen werden können. Dabei wird die Patientenhistorie besonders für den Fall mit einbezogen, dass mit einem für mindestens eine vorhergehende Dialysesitzung als bestmöglich bestimmten Substitutionsziel bei einem Patienten Komplikationen aufgetreten sind, das für die anstehende Dialysesitzung ermittelte bestmögliche Substitutionsziel unter diesen als kritisch erkannten Wert herabgesetzt wird, falls es gleich oder größer als dieser kritische Wert ist.

In einer bevorzugten Ausführungsform ist die Speichereinheit mit einem Bildschirm verbunden, über den die identifizierte Information angezeigt wird. Diese Ausführungsform ist vorteilhaft, weil das bedienende Personal so bequem, schnell und sicher Informationen über die Eigenschaften und den Zustand der Blutbehandlungseinheit bzw. des Blutbehandlungsfilters abrufen kann sowie den Effekt von Steuerbefehlen leicht verifizieren oder verfolgen kann. Das bedienende Personal lässt nach dem Einschalten der Blutbehandlungseinheit erfindungsgemäß die Referenzlösung durch das System laufen und ruft über den Monitor die systemrelevanten Informationen ab, d.h. besonders Informationen über den Filtertyp, Zustand des Filters und des Systems und über die Bestimmung des bestmöglichen Substitutionsziels ab. Diese Bestimmungen des Gerätezustands und des bestmöglichen Substitutionsziels sind keine Behandlungsmethoden, da sie unabhängig vom Patienten ablaufen bzw. durchgeführt werden. Die Einstellung der optimalen Behandlungsparameter geschieht mit einer Referenzlösung und nicht mit Patientenblut; somit ist die Bestimmung des bestmöglichen Substitutionsziels keine Methode zur Behandlung. Damit beruht auch die optionale Anpassung des bestmöglichen Substitutionsziels an veränderte Bedingungen während der Dialysebehandlung auf einem Verfahrensschritt, der ohne Patientenmaterial abläuft. Somit ist auch das Verfahren, dass eine Variation der Behandlungsparameter wie der Blutflussrate oder Dialyseflüssigkeitsflussrate während der Behandlung vorsieht, keine reine Behandlungsmethode.

Der auf der Speichereinheit hinterlegte Druckmesswert kann aus einer oder mehrerer vorherigen Sitzungen von einem bestimmten Patienten mit diesem spezifischen Dialysegerät stammen. Es können gegebenenfalls aber auch Messwerte dieses Patienten von anderen Dialysegeräten verwendet werden, gleichfalls Messwerte von diesem spezifischen Dialysegerät, die nicht patientenspezifisch sind. Es kann sich aber auch aus der Literatur bekannte Richtwerte oder gerätetypische Spezifikationen des Herstellers des Dialysegerätes handeln.

Mit "unmittelbar" ist gemeint, dass zwischen dem Drucksensor und dem genannten Bauteil kein weiteres Bauteil liegt. Die tatsächliche Entfernung zwischen dem Drucksensor und dem genannten Bauteil ist hierbei nicht ausschlaggebend, sondern nur, dass der Drucksensor und das genannte Bauteil nicht durch ein weiteres dazwischen liegendes Bauteil getrennt sind. Erfindungsgemäß liegen zwei Drucksensoren nie unmittelbar hintereinander, also ohne ein weiteres Bauteil zwischen den Drucksensoren. Zudem verwendet die vorliegende Erfindung keinen Drucksensor im Blutkreislauf zwischen Patient ☺ und Pumpe P, weil derartige Drucksensoren zur Überwachung des Patienten dienen und zur Überwachung von Systemänderungen in dem Tangentialflussfilters TFF nicht geeignet sind.

In weiteren bevorzugten Ausführungsformen kann jedoch blutseitig ein Blasenfänger zwischen dem Drucksensor und dem Bauteil angeordnet sein, und/oder auf der Dialysatseite ein Filter. Das zuvor beschriebene Grundprinzip wird aber dadurch nicht in Frage gestellt.

Die Begriffe "vorgeschaltet" und "nachgeschaltet" sind mit Bezug auf die Flussrichtung zu verstehen. Ist ein Drucksensor "vorgeschaltet", so liegt er in Flussrichtung vor dem Bauteil, d.h. das Blut oder die Dialyseflüssigkeit durchläuft zuerst den Drucksensor und dann das Bauteil. Ist ein Drucksensor "nachgeschaltet", dann liegt er in Flussrichtung hinter dem Bauteil, d.h. das Blut oder die Dialyseflüssigkeit durchläuft zuerst das Bauteil und dann den Drucksensor. Die Flussrichtung kann im Blutkreislauf und im Dialysekreislauf gegensätzlich zueinander sein.

Die erfindungsgemäßen Vorrichtungen zur Behandlung von Blut können weiterhin eine Ultrafiltrationspumpe UFP, ein Bilanzkammersystem BK und/oder eine Einheit zum Auswerten der gemessenen Druckmesswerte umfassen. Die Ultrafiltrationspumpe wird für die kontinuierlich geregelte Ultrafiltration benötigt und entnimmt dem geschlossenen System eine genau eingestellte Flüssigkeitsmenge. Dieselbe Menge, die dem geschlossenen Dialysatkreislauf entnommen wird, wird im Tangentialflussfilter TFF dem Blut mittels Unterdruck entzogen. Für die Bilanzierung der ein- und ausgehenden Flüsse sorgt die Bilanzkammer BK, somit wird gewährleistet, dass dem Patienten keine Flüssigkeit unbeabsichtigt entzogen oder zugeführt wird. Die Bilanzkammer kann dabei durch eine flexible Trennwand in zwei Kammerhälften unterteilt werden, die wechselseitig mit dem Ultrafiltrat befüllt werden, das dem Dialysatkreislauf entzogen wird, wobei der Inhalt der jeweils anderen Kammerhälfte verworfen wird. Optional umfasst die erfindungsgemäße Vorrichtung weiterhin eine Tropfkammer. Die Tropfkammer hilft, das Eindringen von Luft in die stromabwärtigen Schläuche zu verhindern, indem eine Flüssigkeitsschicht als eine Luftsperre am Boden der Tropfkammer fungiert.

Die Drucksensoren ([PB1], [PB2], [PD1] und [PD2]) sind vorteilhaft dadurch charakterisiert, dass sie eine Abtastrate von mindestens 20 Hz haben. Eine Abtastrate von 20 Hz bedeutet, dass eine Druckmessung pro Drucksensor 20 mal pro Sekunde erfolgt.

Die Begriffe "dialysatseitig" und "blutseitig" beschreiben die beiden Kreisläufe, die bei der Tangentialflussfiltration, in der Regel im Gegenstromprinzip, aber ggf. auch parallel zum Blutstrom, aneinander vorbeigeführt werden. In die Hohlfasern der Filtermembran werden über einen ersten Flüssigkeitskreislauf Blut/Plasma zugeführt, das sie der Länge nach durchströmt. Durch einen zweiten Flüssigkeitskreislauf wird die Dialyseflüssigkeit auf der Außenseite der Hohlfasern zugeführt. Beide Kreisläufe sind voneinander getrennt und stehen nur über die Filtermembran miteinander in Kontakt.

Erfindungsgemäß kann ein bestmögliches Substitutionsziel in einer Blutbehandlungseinheit dadurch bestimmt werden, dass der Status des Gerätes durch einen Lauf mit einer Referenzlösung ermittelt wird. Die Referenzlösung wird idealerweise standardmäßig bei derselben Blutbehandlungseinheit bzw. demselben Blutbehandlungsfilter verwendet, um die Eigenschaften der Einheit bzw. des Systems zu erfassen. Die Referenzlösung kann beispielsweise und bevorzugt eine physiologische Kochsalzlösung sein; möglich sind aber auch andere Salzlösungen, Glucoselösung, gereinigtes Wasser, oder jede andere Flüssigkeit verwendet werden, die die gleichen Flusseigenschaften wie die Referenzlösung aufweist, oder aus deren Verhalten sich das Verhalten der Referenzlösung ableiten lässt.

Die Referenzlösung kann auch als Testlösung bezeichnet werden, mit der ein Testlauf der Blutbehandlungseinheit durchgeführt werden kann. Erfindungsgemäß kann also ein optimales Substitutionsziel bei der Ultrafiltration von Blut für einen bestimmten Dialysator dadurch bestimmt werden, dass vor der Behandlung eines Patienten eine Testlösung durch das Dialysegerät geleitet wird

Aus den ermittelten Druckmesswerten kann ein auf den Ist-Zustand des Dialysegerätes abgestimmtes Substitutionsziel ermittelt werden. Hierzu wird die Einbeziehung von mindestens zwei Druckmesswerten benötigt, die aus den vorgenannten Druckmesswerten PB1, PB2, PD1, PD2 ausgewählt werden. Erfindungsgemäß können hierfür jeweils Absolutdrücke, relative Drücke, absolute Druckdifferenzen zwischen zwei Druckmesssensoren, relative Druckdifferenzen zwischen zwei Druckmesspunkten, d.h. zwischen zwei Druckmesssensoren, absoluten Druckamplituden, relativen Druckamplituden, Differenzen zwischen den absoluten Druckamplituden an zwei Druckmesspunkten, d.h. an zwei Druckmesssensoren oder Differenzen zwischen den relativen Druckamplituden an zwei Druckmesspunkten, d.h. an zwei Druckmesssensoren oder eine Kombination davon als Messsignal zu Grunde gelegt werden. Dieses optimale Substitutionsziel gibt diejenige Menge der Substitutionslösung an, die dem Patienten zur Aufrechterhaltung seines Blutvolumens und damit seiner Homöostase zugeführt werden muß. Zur Ermittlung des optimalen Substitutionsziels müssen die einzubeziehenden Druckmesswerte an eine zentrale Recheneinheit ausgegeben werden. In dieser zentralen Recheneinheit werden die eingehenden Messwerte derart verarbeitet, dass beispielweise ein guter Impulsübertrag über die Membran eine Vorraussetzung für ein hohes Substitutionsvolumen ist und somit einen entscheidenden Faktor darstellt. Ein auffällig hoher Wert für PB1, beispielsweise verursacht durch eine sehr geringe Durchflussfläche, ist ein Hinweis darauf, keine maximalen Substitutionsvolumina auszuwählen, da hohe Substituatflüsse bei der post-Dilution bedeuten, dass im Filter eine verstärkte Hämokonzentration auftritt und somit PB1 noch weiter steigt. Dies kann im Extremfall zu Druckwarnungen /alarmen führen.

Derartige Daten werden während der Testmessungen erhoben und ihnen sowohl ein Wert für das Ausmaß des Charakteristikums sowie ein Wert für die Wichtigkeit zugeordnet. Aus der Vielzahl dieser Informationen wird so das Substitutionsziel für die Behandlung errechnet. Diese Berechnung kann beispielsweise durch eine Fuzzy-Logic geschehen oder im simplen Fall wird auf der Speichereinheit für jede erdenkliche Kombination von Werte ein Substitutionsziel hinterlegt.

Das derart ermittelte optimale Substitutionsziel kann über die zentrale Recheneinheit angezeigt werden. Dadurch kann das behandelnde Personal geeignete Vorbereitungen treffen, um wenn nötig für die voraussichtliche Behandlungsdauer der nachfolgenden Dialysesitzung genügend Substitutionslösung bereitzustellen. Die Ausgabe kann über eine Anzeige an dem Dialysegerät selber oder an einer dezentralen Anzeige erfolgen. In weiteren bevorzugten Ausführungsformen wird das ermittelte optimale Substitutionsziel automatisch an das Dialysegerät weitergeleitet, wodurch an diesem die nötigen Einstellungen getroffen werden, um das optimale Substitutionsziel erreichen zu können. In bevorzugten Ausführungsformen ist dies lediglich das Substitutionsziel. Dieser Vorgang kann auch automatisch erfolgen.

In einer weiteren bevorzugten Ausführungsform können die gemessenen Werte mit aus der Speichereinheit hinterlegten Patientendaten, verglichen werden und entsprechende Maßnahmen ergriffen werden. D.h. durch Vergleich mit Werten aus besonders gut verlaufenden Behandlungen können entsprechend verwendetem Filter und behandeltem Patient oder Patientengruppe das bestmöglich erreichbare Substitutionsziel ermittelt werden.

In weiteren Ausführungsformen kann das optimale Substitutionsziel in den ersten Minuten einer Dialysesitzung direkt aus dem Blutfluss des Patienten ermittelt werden. Ein Vorlauf mit einer Testlösung ist bei diesen Ausführungsformen nicht nötig oder vorgesehen. Die Ermittlung des optimalen Substitutionsziels und die Umsetzung des Ergebnisses erfolgt analog zu den zuvor beschriebenen Ausführungsformen.

Das Verfahren zur Bestimmung des optimalen Substitutionsziels ist somit wesentlich einfacher als herkömmliche Verfahren, bei denen meistens erst mindestens ein patientenspezifischer Blutparameter wie der Hämatokrit und Proteinkonzentration im Labor bestimmt werden muß oder eher vage Anhaltspunkte wie die Patientenhistorie herangezogen wird. Das Verfahren ist hier wesentlich zielgerichteter, da der aktuelle Zustand des Dialysators der entscheidende Indikator für den Verlauf der nachfolgenden Dialysesitzung ist. Hierbei werden Messwerte verwendet, die zum Betrieb des Dialysators sowieso erhoben werden. Dies bedeutet, dass kein größerer zeitlicher Vorlauf in Kauf genommen werden muß und keine Zusatzkosten wie z.B. für eine Laboranalyse anfallen. Zudem kann das Substitutionsziel während des Dialysevorgangs angepasst werden, wenn Veränderungen im Flussverhalten darauf hindeuten, dass das zuvor berechnete optimale Substitutionsziel nicht mehr erreicht werden kann. Hierbei wird auf dieselben Messverfahren zurückgegriffen wie für die Bestimmung der Anfangseinstellung. Daher können Anpassungen zielgenauer erfolgen, da die Messwerte eine Vergleichbarkeit garantieren.

In weiteren bevorzugten Ausführungsformen können jedoch die derart ermittelten Druckmesswerte mit zuvor erhobenen Messwerten für Blutfluss, Hämatokrit, Proteinkonzentration und Patientenhistorie in der zentralen Recheneinheit kombiniert werden.

Ein weiterer Vorteil der Verwendung von Kochsalzlösung als Testlösung ist, dass dies zugleich der Reinigung des Dialysators dient, da ein guter Teil der Rückstände aus der vorhergehenden Behandlung mit ausgewaschen wird. Durch ein derartiges Verfahren können zwei Arbeitsschritte in einem durchgeführt werden. Aus Kostengründen hat es sich - besonders in den USA - in letzter Zeit vermehrt durchgesetzt, den Dialysefilter zu reinigen, anstatt pro Patient und Sitzung einen neuen zu nehmen. In diesem Fall kann die zur Reinigung verwendete Lösung gleichzeitig auch die Referenzlösung zur Evaluierung der Filtereigenschaften sein.

Das errechnete Substitutionsziel kann während der Behandlung in vordefinierten Abständen überprüft und angepasst werden.

In bevorzugten Ausführungsformen werden die erwähnten Druckparameter kontinuierlich überwacht. Bei Überschreiten eines zuvor festgelegten Grenzwerts wird daraufhin eine Anpassung des Substitutionsflusses eingeleitet. Die Verrechnung der eingehenden Druckmesswerte erfolgt nach demselben Verfahren wie zuvor beschrieben. Bei der kontinuierlichen Anpassung des Substitutionsziels kann dies ebenfalls über Bedienungspersonal und/oder eine automatische Anpassung des Substituatflusses erfolgen.

Charakteristischerweise wirken sich die Mechanismen, die einen Einfluss auf die Filtereigenschaften haben wie beispielsweise die Proteinkonzentrationspolarisation, die Sekundärmembran (der Besatz der Filtermembran mit Blutzellen wie beispielsweise Thrombozyten und weiteren Blutbestandteilen) oder das Clotting, negativ auf das Filtrationspotential. In diesen Fällen muß das Substitutionsziel nach unten angepasst werden, d.h. es kommt zu einer Verringerung des Substitutionsflusses.

Da dies die Qualität der Behandlung beeinträchtigen kann, ist es notwendig eine weitere Möglichkeit in Betracht zu ziehen, das Erhalten bzw. Wiederherstellen der Filtereigenschaften während der Behandlung.

Sollte ein zu hoher Hämatokrit als Ursache für ein Clotting des Dialysefilters festgestellt oder vermutet werden, besteht erfindungsgemäß die Option, während des Dialysevorgangs eine Blutverdünnung vorzunehmen, um einer weiteren Verschlechterung der Dialyseleistung entgegenzuwirken. Typischerweise findet eine Gabe eines Anti-Koagulantiums wie Heparin bzw. Heparansulfat statt, es kann aber auch jedes weitere zugelassene blutverdünnende Mittel oder Anti-Koagulantium wie beispielsweise Marcomar oder Clopidogrel verwendet werden. In bevorzugten Ausführungsformen befindet sich zusätzlich am Dialysegerät ein Reservoir, das mit einer gebrauchsfertigen Lösung des Blutverdünnungsmittels befüllt ist. Bei Indikation kann aus diesem Reservoir mittels eines Perfusors über ein Ventil die Lösung mit dem Blutverdünnungsmittel in festen Mengen pro Zeiteinheit oder alternativ als Einmal-Bolusgabe in den Blutkreislauf zugeführt werden. Die Bedienung bzw. Aktivierung dieses Ventils erfolgt manuell durch das Dialysepersonal oder aber durch ein automatisches Überwachungssystem, das von der zentralen Recheneinheit kontrolliert wird.

In weiteren bevorzugten Ausführungsformen besteht alternativ dazu ein Port zu dem Blutfluss durch den Dialysator, an dem das blutverdünnende Mittel beispielsweise über eine Infusionsschlauch infundiert wird oder aber als Einmal-Bolusgabe injiziert wird.

Im umgekehrten Fall, dass ein erniedrigter blutseitiger Flusswiderstand (PBE-PV) gemessen wird und dies mit einem niedrigen Hämatokrit des Patienten zusammenfällt, kann dementsprechend eine Reduktion der Heparindosierung vorgeschlagen oder eingeleitet werden.

Wird bei der kontinuierlichen oder sequenziellen Überprüfung der Filtereigenschaften festgestellt, dass sich das Leistungspotential verschlechtert hat, können folgende Gegenmaßnahmen eingeleitet werden:

  i. Antikoagulation des Blutes durch Zugabe eines Antikoagulantiums wie beispielsweise Heparin
  ii. Spülung des Dialysators mit einer Kochsalzlösung oder einer wässrigen Lösung von Kochsalz und Zitrat
  iii. Dialysat- und/oder blutseitige Druckpulsation wird eingeleitet
  iv. Blutverdünnung vor dem Dialysator beispielsweise durch eine physiologische Kochsalzlösung

v. Dialysatorwechsel

Es hat sich als besonders vorteilhaft herausgestellt, ein Ultrafiltrationsmuster zu wählen, bei dem die Geschwindigkeit, mit der die Ultrafiltrationsrate bzw. die Substitution am Anfang der Therapie gesteigert wird, gering gehalten wird. Dies ist umso bedeutender, je höher der zu erreichende Endwert (das Substitutionsziel) ist.

Diese Änderungsgeschwindigkeit bzw. das Differential der Ultrafiltrationsrate der dialysatseitigen Drücke bzw. deren Druckamplituden absolut oder relativ zu den blutseitigen kann als Kontrollparameter verwendet werden. Sollten sich diese zu schnell ändern bzw. das Differential zu groß werden, wird auch die Steigung der UF-Rampe (Änderungsgeschwindigkeit der UF-Rate) angepasst werden. Hierbei hat sich gezeigt, dass niedrigere Steigungsraten vorteilhaft sind, da so die Ausbildung einer Sekundärmembran weniger starke Auswirkungen auf die Permeabilität hat.

Mit Einbeziehung der Patientenhistorie in die Bestimmung des jeweiligen Substitutionsziels ist gemeint, dass der behandelnden Person und/oder der zentralen Recheneinheit eine Information zur Verfügung gestellt wird, sollte die gewählte Ultrafiltrationsrate bei den vorherigen Dialysesitzungen Komplikationen verursacht haben. Es sollte angestrebt werden, unterhalb dieser als zu hoch befundenen Ultrafiltrationsrate zu bleiben. Sollte bei der Berechnung des optimalen Substitutionsziels ein Wert herauskommen, der oberhalb dieser kritischen Ultrafiltrationsrate liegt, ist es bevorzugt, dass das optimale Substitutionsziel dementsprechend nach unten angepasst wird. In bevorzugten Ausführungsformen kann von der zentralen Recheneinheit ein Vorschlag ausgegeben bzw. angezeigt werden, die einen Behandlungsvorschlag bzw. ein neues Substitutionsziel enthält, wobei die besagte Patientenhistorie in Betracht gezogen wird.

Ein Blutdruckabfall oder hypotensive Phasen sind eine der häufigsten Komplikationen bei Dialysepatienten. Daher kann der Blutdruck dieser Patienten während der Dialyse überwacht werden. Wird nun ein Blutdruckabfall oder eine hypotensive Phase diagnostiziert, kann erfindungsgemäß die Ultrafiltrationsrate und damit der Fluss der Substitutionslösung durch das Dialysepersonal oder automatisch reduziert werden oder eine solche Maßnahme von der zentralen Recheneinheit vorgeschlagen werden. Desweiteren ist es möglich, vorübergehend in den Hämofiltrationsmodus zu wechseln oder einen solchen Wechsel vorzuschlagen. Auch eine Kombination aus beiden ist möglich.

Für alle Signale der jeweiligen Druckmeßsensoren bzw. die von ihnen abgeleiteten Kenngrößen können im voraus Toleranzintervalle festgelegt werden, innerhalb derer die Druckmesswerte schwanken können, ohne als kritische Abweichung eingestuft zu werden. Die Messtoleranz des Sensors hängt dabei von verschiedenen Quellen ab, nämlich zum einen von der Toleranz des Sensors selbst, die im Allgemeinen vom Hersteller angegeben wird, aber auch von gewissen Umgebungsbedingungen abhängt; weiterhin von der Variabilität des Mediums, also Temperatur und Zusammensetzung der strömenden Flüssigkeiten; und Veränderungen des Systems, die z.B. durch Verunreinigungen oder Ablagerungen an den Sensoren entstehen können. Der Toleranzbereich berücksichtigt sowohl eine gewisse Messwerte-Unsicherheit als auch empirische Erfahrung, in welchem Maße Abweichungen vom Sollwert keine Auswirkung auf die Funktion des Systems haben. Das bedeutet, dass die Abweichung bzw. Schwankung des Wertes oder der Kenngröße vom Sollzustand noch keine regulierende Maßnahme notwendig macht. Befinden sich die gemessenen Druckmesswerte oder Kenngrößen also innerhalb des Toleranzbereichs, sind keine Regulierungen wie Veränderung der Flussrate von Blut, Dialyseflüssigkeit oder Substituat notwendig. Beispielsweise können diese Toleranzintervalle an der zentralen Recheneinheit durch Eingabe eingestellt werden oder auf dieser hinterlegt worden sein oder aus einer vorgegebenen Liste über einen Menübefehl auswählbar sein. Bei der Festlegung eines solchen Toleranzintervalls können die während einer Behandlungssitzung ermittelten Druckmesswerte optional in Kombination mit dem Blutfluss, dem Hämatokrit des Patienten, der Gesamtproteinkonzentration des Patienten oder der Patientenhistorie oder einer Kombination davon verwendet werden. Erfahrungsgemäß werden Toleranzintervalle im Bereich von 0,1% bis 100 % bevorzugt. Weiter bevorzugt sind Werte zwischen 5% und 70% und am Bevorzugtesten Werte zwischen 15% und 50%. Hat man allerdings sehr niedrige Werte der jeweiligen Behandlungsparameter, kann ein relatives Toleranzintervall von 100% nicht zielführend sein, weshalb ein zusätzliches Toleranzintervall über Absolutwerte definiert wird. Dieses stellt den Minimalwert für die Toleranz dar.

Die Toleranzbereiche können sich dabei bevorzugt auf Werte beziehen, die an demselben Sensor gemessen werden. Der Vergleich des gemessenen Werts erfolgt mit einem korrespondierenden Toleranzintervall, das auf der Speichereinheit hinterlegt wurde. Befindet sich der gemessene Wert innerhalb des Toleranzbereichs, ist keine Regulierung erforderlich. Befindet sich der gemessene Wert außerhalb des Toleranzbereichs, wird ein Steuerbefehl zur Veränderung z.B. der Blutflussrate gegeben. Für den Vergleich der Werte mit dem Toleranzbereich können auch Quotienten, Differenzen, Summen oder Produkte mehrerer, an jeweils verschiedenen Sensoren gemessenen Werte genutzt werden. Besonders bevorzugt ist dabei die Benutzung von Werten, die an zwei Sensoren gemessen wurden. Dabei werden vorzugsweise Differenzen zwischen Sensorenwerten berechnet, die sich auf derselben Seite der Filtermembran befinden, also beide blutseitig oder dialysatseitig sind. Es ist jedoch auch bevorzugt, wenn Differenzen berechnet werden, die sich auf zwei verschiedenen Seiten der Filtermembran befinden, also einer blutseitig und einer dialysatseitig ist.

Bei einem Verlassen des Toleranzbereichs (Toleranzintervalls) von PD1 oder PD2 oder deren Druckamplituden absolut oder relativ durch sinkende Werte (in Richtung kleinerer Werte) kann beispielsweise eine Pulsation auf der Dialysat- oder Blutseite gestartet oder verstärkt werden oder die aktuelle Ultrafiltrationsrate und der Substitutionsfluss kann reduziert oder diese Maßnahmen vorgeschlagen werden. Bei einem Verlassen des Toleranzintervalls von PD1 oder PD2 oder deren Druckamplituden absolut oder relativ in Richtung steigender Werte kann hingegen eine Pulsation auf der Dialysat- oder Blutseite gestoppt oder verringert werden oder die aktuelle Ultrafiltrationsrate und der Substitutionsfluss reduziert oder diese Maßnahmen vorgeschlagen werden. Dabei vergleicht die zentrale Recheneinheit die gemessenen Druckmesswerte mit entsprechenden Referenzdruckmesswerten unter Berücksichtigung eines Toleranzbereichs und schlägt bei Abweichungen über den Toleranzbereich Maßnahmen vor, um bei festgelegter Behandlungszeit das Substitutionsziel bestmöglichst zu erreichen oder bei festgelegtem Substitutionsziel die Behandlungsdauer möglichst wenig zu verändern, in der Regel möglichst wenig zu verlängern.

Bei einem Verlassen des Toleranzbereichs der blutseitigen Drücke durch einen erniedrigten Widerstand in Blutflussrichtung kann eine Verringerung der Spülrate, eine geringere Blutverdünnung oder eine Änderung des Blutflusses vorgeschlagen oder eingeleitet werden.

Ein schematisches Flusssystem mit den folgenden Komponenten ist in Fig. 1 abgebildet: Eine Pumpe P, vorzugsweise eine peristaltische Pumpe, erzeugt den gewünschten Fluss im extrakorporalen Kreislauf. Im Blutkreislauf, mit gestrichelten Linien dargestellt, durchläuft das Blut vom Patienten ☺ aus zunächst die Pumpe P, dann den ersten blutseitigen Drucksensor [PB1], den Tangentialflussfilter TFF und bevor es in den Patienten ☺ zurückfließt noch einen weiteren Drucksensor [PB2]. Im Gegenstromprinzip wird Dialyseflüssigkeit durch den Filter TFF gepumpt. Im Dialysekreislauf, mit durchgehenden Linien dargestellt, befindet sich in Flussrichtung vor dem Filter TFF der erste dialysatseitige Drucksensor [PD1] und hinter dem Filter TFF der zweite Drucksensor [PD2]. Für die Bilanzierung der ein- und ausgehenden Flüsse sorgt die Bilanzkammer BK, somit wird gewährleistet, dass dem Patienten ☺ keine Flüssigkeit unbeabsichtigt entzogen oder zugeführt wird. Der für die Therapie verordnete Gewichtsverlust wird durch die Ultrafiltrationspumpe UFP erzeugt, die das Bilanzkammersystem BK umgeht.

**Figurenbeschreibung**

**Figur 1**:    Schema für ein erfindungstypisches Flusssystem
**Figur 2**:    Vergleich verschiedener Filter. A und B sind Druckverläufe des high-flux-Filters, C und D des low-flux-Filters, wobei die Filter die gleiche Größe haben.
**Figur 3**:    Matrix zur Ermittlung des Faktors p

**Beispiele**

**Beispiel 1:**

Therapieverlauf ohne Notwendigkeit einer Nachregulierung

a.

i) Die Druckmessung an zwei Drucksensoren hat ergeben, dass der Filter einen sehr hohen Permeabilitätswert aufweist und somit für hohe transmembrane Flüsse geeignet ist. p ergibt sich zu 1.
ii) Neben dem Blutfluss (300 ml/min) ist keine weitere Größe bekannt.
iii) Es wird aus der Formel Qsub = [(1/3*BF*T)-WL]*p (1) eine Substitutionsrate von 100 ml/min bestimmt und somit bei einer Behandlungsdauer von 240 min ein Substitutionsziel von 24 l berechnet.
iv) Automatische Wahl des Substitutionszieles nach beschriebener Vorgehansweise zu 24 l.

b. Start der Therapie,
c. Sequenzielle oder kontinuierliche Überwachung der Drücke und optional daraus abgeleiteter Parameter,
d. nach störungsfreiem Behandlungsverlauf Erreichen des Substitutionsziels von 24 l am Ende der Dialysesitzung nach 240 Minuten.

**Beispiel 2:**

Therapieverlauf mit Nachregulierung bei sequenzieller Überwachung

a.

i) Die Druckmessung an zwei Drucksensoren hat ergeben, dass der Filter einen sehr hohen Permeabilitätswert aufweist und somit für hohe transmembrane Flüsse geeignet ist. p ergibt sich zu 1.

ii) Neben dem Blutfluss (300 ml/min) ist keine weitere Größe bekannt.

iii) Es wird aus der Formel $Qsub = [(1/3*BF*T)-WL]*p$ (1) eine Substitutionsrate von 100 ml/min bestimmt und somit bei einer Behandlungsdauer von 240 min ein Substitutionsziel von 24 l.

iv) Automatische Wahl des Substitutionszieles nach beschriebener Vergehensweise zu 24 l.

b. Start der Therapie,

c. Sequenzielle Überwachung der Parameter ergibt, dass der Quotient $A_{PD2}/A_{PB1}$ auf weniger als 70% des Startwertes gefallen ist. Das zuvor festgelegte Toleranzintervall von 30% ist damit überschritten.

d. Der Substituatfluss wird um 10% reduziert und die Überwachung wird resettet.

e. Ende der Therapie: die Kontrollparameter haben sich aufgrund der Nachregelung der Flußraten in den gewünschten Grenzen bewegt. Insgesamt wurde während der Dialysesitzung von 240 Minuten so ein Substitutionsvolumen von 22 l erreicht.

**Beispiel 3:**

Therapieverlauf mit Nachregulierung bei kontinuierlicher Überwachung

a.

i) Die Druckmessung an zwei Drucksensoren hat ergeben, dass der Filter einen sehr hohen Permeabilitätswert aufweist und somit für hohe transmembrane Flüsse geeignet ist. p ergibt sich zu 1.

ii) Bei einem Blutfluss von 300 ml/min ist der Eingangshämatokrit 30% und der maximale Hämatokrit ist 47%.

iii) Es wird aus $Qsub = [BF*\{1-[Hct(ein)/Hct(max)]\}-WL]*p$ bei einer Blutflussrate von 300 ml/min, einem Eingangshämatokrit von 30% und einem maximalen Hämatokrit von 47% ein Substitutionsziel von 27 l bei einer vierstündigen Therapie berechnet.

b. Start der Therapie,

c. Kontinuierliche Überwachung der Drücke an einem blutseitigen und einem dialysatseitigen Drucksensor,

d. es wird festgestellt, dass das Substitutionsziel am Ende der 4-stündigen Blutbehandlung nicht erreicht werden wird. PD2 ist zu stark abgefallen und hat das zuvor festgelegte Toleranzintervall von 35% nach unten verlassen.

e. es erfolgt eine Reduktion der Substituatflussrate um 10% und der Druckverlauf liegt wieder innerhalb des Toleranzinteravalls.

f. weitere kontinuierliche Überwachung der Drücke

g. Ende der Therapie: die Kontrollparameter haben sich aufgrund der Nachregelung der Flußraten in den gewünschten Grenzen bewegt. Insgesamt wurde so nach 4 Stunden ein Substitutionsvolumen von 24 l erzielt.

**Beispiel 4:**

Therapieverlauf mit Nachregulierung bei kontinuierlicher Überwachung

a.

i) Die Druckmessung an drei Drucksensoren hat ergeben, dass der Filter einen sehr hohen Permeabilitätswert aufweist und somit für hohe transmembrane Flüsse geeignet ist. p ergibt sich zu 1.

ii) Bei einem Blutfluss von 400 ml/min ist der Eingangshämatokrit 30% und der maximale Hämatokrit ist 40%.

iii) Es wird aus $Qsub = [BF*\{1-[Hct(ein)/Hct(max)]\}-WL]*p$ bei einer Blutflussrate von 400 ml/min, einem Eingangshämatokrit von 30% und einem maximalen Hämatokrit von 40% ein Substitutionsziel von 26 l bei einer vierstündigen Therapie berechnet.

b. Automatische Wahl des Substitutionszieles nach beschriebener Vorgehensweise zu 26 l,

c. Start der Therapie,

d. Kontinuierliche Überwachung der Drücke an drei Drucksensoren,

e. es wird anhand des Frequenzspektrums der Druckmesswerte an den drei Drucksensoren festgestellt, dass das Substitutionsziel am Ende der fünfstündigen Blutbehandlung nicht erreicht werden wird, weil zwei der drei gemessenen Druckmesswerte mehr als 25% von den hinterlegten Referenzdruckmesswerten abweichen,

f. es erfolgt eine Nachregulierung der Blutfußrate und der Substituatflußrate,

g. nach einmaliger Nachregulierung liegen die gemessenen drei Druckmesswerte innerhalb des Toleranzbereichs von 25% der Referenzdruckmesswerte,

h. Ende der Therapie: die Dialyseparameter haben sich aufgrund der Nachregelung in den gewünschten Grenzen der Referenzdruckmesswerte bewegt. Insgesamt wurde nach 5-stündiger Blutbehandlung so ein Substitutionsziel von 25,5 l erreicht

**Beispiel 5:**

Bestimmung eines konkreten Filtertyps über die Messung von Druckmesswerten an mindestens zwei Drucksensoren mit einer Referenzlösung

    a.

        i) Anhand der Auswertung der Druckamplituden $A_{PB2}/A_{PB1}$ und $A_{PD2}/A_{PB1}$ wird der Filter mittels Vergleich mit einer Referenztabelle identifiziert und ein p=1 zugeordnet.
        ii) Neben dem Blutfluss (300 ml/min) ist keine weitere Größe bekannt.
        iii) Es wird aus der Formel $O_{sub}$ = [(1/3*BF*T)-WL]*p (1)eine Substitutionsrate von 100 ml/min bestimmt und somit bei einer Behandlungsdauer von 240 min ein Substitutionsziel von 24 l berechnet.
        iv) Automatische Wahl des Substitutionszieles nach beschriebener Vergehensweise zu 24 l.

    b. Start der Therapie,
    c. Sequenzielle oder kontinuierliche Überwachung der Drücke und optional daraus abgeleiteter Parameter,
    d. nach störungsfreiem Behandlungsverlauf Erreichen des Substitutionsziels von 24 l am Ende der Dialysesitzung nach 240 Minuten.

**Beispiel 6**

Erkennen eines für eine HOF-Therapie ungeeigneten Filters

    a.

        i) Beim Anfahren der Ultrafiltration von 0 ml/h auf 2000 ml/h fällt der PD2 um mehr als 50 mmHg.
        ii) Dies würde bedeuten, dass der Filter für eine HOF-Therapie ungeeignet ist (p<0,5).

    b. Dem Anwender wird eine Warnung ausgegeben, dass eine HOF-Therapie mit diesem Filter nicht durchführbar ist.

**Referenzzeichenliste:**

PB1    Druck gemessen am Drucksensor [PB1]
PB2    Druck gemessen am Drucksensor [PB2]
PD1    Druck gemessen am Drucksensor [PD1]
PD2    Druck gemessen am Drucksensor [PD2]
[PB1, PB2, PD1, PD2]:    Drucksensoren
[PB1]    Blutseitiger Drucksensor vor dem Tangentialflussfilter
[PB2]    Blutseitiger Drucksensor hinter dem Tangentialflussfilter
[PD1]    Dialysatseitiger Drucksensor vor dem Tangentialflussfilter
[PD2]    Dialysatseitiger Drucksensor hinter dem Tangentialflussfilter
$A_{PB1}$    Amplitude des am Drucksensor [PB1] gemessenen Drucks PB1
$A_{PB2}$    Amplitude des am Drucksensor [PB2] gemessenen Drucks PB2

$A_{PD1}$ ☺    Amplitude des am Drucksensor [PD1] gemessenen Drucks PD1
$A_{PD2}$    Amplitude des am Drucksensor [PD2] gemessenen Drucks PD2
UFP    Ultrafiltrationspumpe
P    Pumpe
TFF    Tangentialflussfilter (Dialysator)
    Patient
BK    Bilanzkammer

**Patentansprüche**

1. Blutbehandlungseinheit mit einem Dialysator, mindestens zwei Drucksensoren, einer zentralen Recheneinheit und einer Speichereinheit, wobei die zentrale Recheneinheit und die Speichereinheit dazu angepasst sind, um folgendes Verfahren auszuführen:

   a) Bestimmung der Filtereigenschaften vor der eigentlichen Dialysesitzung durch Ermittlung von mindestens zwei Druckmesswerten an mindestens zwei Drucksensoren innerhalb der Blutbehandlungseinheit mit einer Referenzlösung,
   b) Vergleich der gemessenen Druckmesswerte mit auf der Speichereinheit hinterlegten Referenzwerten desselben Patienten, welche in früheren Dialysesitzungen erhoben wurden oder mit auf der Speichereinheit hinterlegten Referenzwerten einer Patientengruppe,
   c) Berechnung eines Substitutionsziels auf Grundlage der Werte gemäß Schritt a) und Schritt b),
   d) Einstellung der Flussrate von Blut, Dialysat und/oder Substituat bei Abweichung von mindestens einem gemessenen Druckmesswert von einem auf der Speichereinheit hinterlegten Referenzdruckmesswert, um bei festgelegter Behandlungszeit das Substitutionsziel bestmöglichst zu erreichen oder bei festgelegtem Substitutionsziel die Behandlungsdauer möglichst wenig zu verändern.

2. Blutbehandlungseinheit gemäß Anspruch 1, wobei das Verfahren des Weiteren die Schritte e) - g) umfasst:

   e) Messen von Druckmesswerten an mindestens zwei Drucksensoren während einer Blutbehandlung,
   f) Vergleich der gemäß Schritt e) gemessenen Druckmesswerte mit auf der Speichereinheit hinterlegten Referenzdruckmesswerten, und
   g) Einstellung der Flussrate von Blut, Dialysat und/oder Substituat bei Abweichung von mindestens einem gemessenen Druckmesswert von dem Referenzdruckmesswert, um bei festgelegter Behandlungszeit das Substitutionsziel bestmöglichst zu erreichen oder bei festgelegtem Substitutionsziel die Behandlungsdauer möglichst wenig zu verändern.

3. Blutbehandlungseinheit gemäß Anspruch 1 oder 2, wobei die Bestimmung der Filtereigenschaften gemäß Schritt a) die Bestimmung des Filtertyps umfasst, sofern Referenzwerte des Filtertyps auf der Speichereinheit hinterlegt sind.

4. Blutbehandlungseinheit gemäß einem der Ansprüche 1, 2 oder 3, wobei das Substitutionsziel über die Auswertung von Druckmesswerten gemessen an drei Drucksensoren bestimmt wird.

5. Blutbehandlungseinheit gemäß einem der Ansprüche 1, 2 oder 3, wobei das Substitutionsziel über die Auswertung von Druckmesswerten gemessen an vier Drucksensoren bestimmt wird.

6. Blutbehandlungseinheit gemäß einem der Ansprüche 1 - 5, wobei die zentrale Recheneinheit die gemessenen Druckmesswerte mit Referenzdruckmesswerten unter Berücksichtigung eines Toleranzbereichs vergleicht und bei Abweichungen über den Toleranzbereich Maßnahmen vorschlägt, um bei festgelegter Behandlungszeit das Substitutionsziel bestmöglichst zu erreichen oder bei festgelegtem Substitutionsziel die Behandlungsdauer möglichst wenig zu verändern.

7. Blutbehandlungseinheit gemäß einem der Ansprüche 1 - 6, wobei die Einstellung der Flussraten von Blut, Dialysat und/oder Substituat automatisch erfolgt.

8. Blutbehandlungseinheit gemäß Anspruch 1 oder 2, wobei die Einstellung der Flussrate von Blut, Dialysat und/oder Substituat gemäß Schritt d) oder g) über den mathematischen Zusammenhang zwischen den gemessenen Druckmesswerten und der Blutflussrate erfolgt.

9. Blutbehandlungseinheit gemäß Anspruch 1 oder 2, wobei die Einstellung der Flussrate von Blut, Dialysat und/oder Substituat gemäß Schritt d) oder g) über den mathematischen Zusammenhang zwischen den gemessenen Druckmesswerten und dem Hämatokrit des Bluts erfolgt.

10. Blutbehandlungseinheit gemäß Anspruch 1 oder 2, wobei die Einstellung der Flussrate von Blut, Dialysat und/oder Substituat gemäß Schritt d) oder g) über den mathematischen Zusammenhang zwischen den gemessenen Druckmesswerten und dem Anteil der einen nicht membrangängigen Blutbestandteile erfolgt.

**11.** Blutbehandlungseinheit gemäß Anspruch 2, wobei die Referenzdruckmesswerte gemäß Schritt f) erhoben wurden bei Blutbehandlungen, in denen das Substitutionsziel während der Behandlungszeit erreicht worden ist.

**Claims**

**1.** Blood treatment unit having a dialysis unit, at least two pressure sensors, a central computation unit and a storage unit, wherein the central computation unit and the storage unit are configured to carry out following method:

a) determination of the filter characteristics prior to the actual dialysis sitting by means of the determination of at least two pressure measurement values at at least two pressure sensors within the blood treatment unit with a reference solution,
b) comparison of the pressure measurement values with reference values, stored in the storage unit, of the same patient, which were gained in earlier dialysis sittings, or with reference values, stored in the storage unit, of a patient group,
c) calculation of a substitution target on the basis of the values according to step a) and step b),
d) setting of the flow rates of blood, dialysate and/or substituate when at least one pressure measurement value differs from a reference pressure measurement value stored in the storage unit, in order in a fixed treatment time to achieve as closely as possible the substitution target or at a fixed substitution target to change as little as possible the treatment duration.

**2.** Blood treatment unit according to claim 1, wherein the method further comprises the steps e) - g):

e) measurement of pressure measurement values at at least two pressure sensors during a blood treatment,
f) comparison of the pressure measurement values according to step e) with reference pressure measurement values stored in the storage unit, and
g) setting of the flow rates of blood, dialysate and/or substituate when at least one pressure measurement value differs from the reference pressure measurement value, in order in a fixed treatment time to achieve as closely as possible the substitution target or at a fixed substitution target to change as little as possible the treatment duration.

**3.** Blood treatment unit according to claim 1 or 2, wherein the determination of the filter characteristics according to step a) comprises the determination of the filter type, inasmuch as reference values for the filter type are stored in the storage unit.

**4.** Blood treatment unit according to any one of the claims 1, 2 or 3, wherein the substitution target is determined by the evaluation of pressure measurement values measured at three pressure sensors.

**5.** Blood treatment unit according to any one of the claims 1, 2 or 3, wherein the substitution target is determined by the evaluation of pressure measurement values measured at four pressure sensors.

**6.** Blood treatment unit according to any one of the claims 1-5, wherein the central computing unit compares the pressure measurement values with reference pressure measurement values taking into account a tolerance range and at deviations above the tolerance range suggests measures in a fixed treatment time to achieve as closely as possible the substitution target or at a fixed substitution target to change as little as possible the treatment duration.

**7.** Blood treatment unit according to any one of the claims 1-6, wherein the setting of the flow rates of blood, dialysate and/or substituate takes place automatically.

**8.** Blood treatment unit according to claim 1 or 2, wherein the setting of the flow rate of blood, dialysate and/or substituate according to step d) or g) is determined by the mathematical relationship between the pressure measurement values and the blood flow rate.

**9.** Blood treatment unit according to claim 1 or 2, wherein the setting of the flow rate of blood, dialysate and/or substituate according to step d) or g) is determined by the mathematical relationship between the pressure measurement values and the haematocrit of the blood.

**10.** Blood treatment unit according to claim 1 or 2, wherein the setting of the flow rate of blood, dialysate and/or substituate according to step d) or g) is determined by the mathematical relationship between the pressure measurement values and the proportion of the blood components which are not membrane-permeable.

11. Blood treatment unit according to claim 2, wherein the reference pressure measurement values according to step f) were gained during blood treatments in which the substitution target was reached during the treatment time.

**Revendications**

1. Unité de traitement du sang avec un dialyseur, avec au moins deux capteurs de pression, avec un ordinateur central et avec une mémoire, dans laquelle l'ordinateur central et la mémoire sont adaptés pour mettre en oeuvre le procédé suivant :

   a) détermination des propriétés de filtre avant la session de dialyse proprement dite en déterminant au moins deux valeurs de mesure de pression au niveau d'au moins deux capteurs de pression à l'intérieur de l'unité de traitement du sang avec une solution de référence,
   b) comparaison des valeurs de mesure de pression mesurées à des valeurs de référence, enregistrées dans la mémoire, du même patient, lesquelles valeurs de référence ont été relevées lors de sessions de dialyse antérieures, ou à des valeurs de référence, enregistrées dans la mémoire, d'un groupe de patient,
   c) calcul d'une cible de substitution sur la base des valeurs selon l'étape a) et l'étape b),
   d) réglage du débit de sang, de dialysat et/ou de produit de substitution en présence d'un écart entre au moins une valeur de mesure de pression mesurée et une valeur de mesure de pression de référence enregistrée dans la mémoire afin d'atteindre le mieux possible la cible de substitution pour un temps de traitement fixé ou de modifier le moins possible la durée de traitement pour une cible de substitution fixée.

2. Unité de traitement du sang selon la revendication 1, dans laquelle le procédé comprend en outre les étapes e) - g) :

   e) mesure de valeurs de mesure de pression au niveau d'au moins deux capteurs de pression pendant un traitement du sang,
   f) comparaison des valeurs de mesure de pression mesurées selon l'étape e) à des valeurs de mesure de pression de référence enregistrées dans la mémoire, et
   g) réglage du débit de sang, de dialysat et/ou de produit de substitution en présence d'un écart entre au moins une valeur de mesure de pression mesurée et la valeur de mesure de pression de référence afin d'atteindre le mieux possible la cible de substitution pour un temps de traitement fixé ou de modifier le moins possible la durée de traitement pour une cible de substitution fixée.

3. Unité de traitement du sang selon la revendication 1 ou 2, dans laquelle la détermination des propriétés de filtre selon l'étape a) comprend la détermination du type de filtre dans la mesure où des valeurs de référence du type de filtre sont mémorisées dans la mémoire.

4. Unité de traitement du sang selon l'une quelconque des revendications 1, 2 ou 3, dans laquelle la cible de substitution est déterminée par l'intermédiaire de l'évaluation de valeurs de mesure de pression mesurées au niveau de trois capteurs de pression.

5. Unité de traitement du sang selon l'une quelconque des revendications 1, 2 ou 3, dans laquelle la cible de substitution est déterminée par l'intermédiaire de l'évaluation de valeurs de mesure de pression mesurées au niveau de quatre capteurs de pression.

6. Unité de traitement du sang selon l'une quelconque des revendications 1-5, dans laquelle l'ordinateur central compare les valeurs de mesure de pression mesurées à des valeurs de mesure de pression de référence en tenant compte d'une plage de tolérance et, en présence d'écarts au-delà de la plage de tolérance, il propose des dispositions pour atteindre le mieux possible la cible de substitution pour un temps de traitement fixé ou modifier le moins possible la durée de traitement pour une cible de substitution fixée.

7. Unité de traitement du sang selon l'une quelconque des revendications 1-6, dans laquelle le réglage du débit de sang, de dialysat et/ou de produit de substitution s'effectue automatiquement.

8. Unité de traitement du sang selon la revendication 1 ou 2, dans laquelle le réglage du débit de sang, de dialysat et/ou de produit de substitution selon l'étape d) ou g) s'effectue par l'intermédiaire de la relation mathématique entre les valeurs de mesure de pression mesurées et le débit du sang.

9. Unité de traitement du sang selon la revendication 1 ou 2, dans laquelle le réglage du débit de sang, de dialysat et/ou de produit de substitution selon l'étape d) ou g) s'effectue par l'intermédiaire de la relation mathématique entre les valeurs de mesure de pression mesurées et l'hématocrite du sang.

10. Unité de traitement du sang selon la revendication 1 ou 2, dans laquelle le réglage du débit de sang, de dialysat et/ou de produit de substitution selon l'étape d) ou g) s'effectue par l'intermédiaire de la relation mathématique entre les valeurs de mesure de pression mesurées et la proportion d'un constituant du sang ne traversant pas la membrane.

11. Unité de traitement du sang selon la revendication 2, dans laquelle les valeurs de mesure de pression de référence selon l'étape f) ont été relevées lors de traitements du sang lors desquels la cible de substitution a été atteinte pendant le temps de traitement.

**Figuren**

**Figur 1**

## Figur 2A

Druckverhalten bei hoher Permeabilität und UF=0

PB2    PB1    PD2

Figur 2B

Druckverhalten bei hoher Permeabilität und UF>0

PB2    PB1    PD2

EP 2 640 439 B1

Figur 2C

Druckverhalten bei niedriger Permeabilität und UF=0

Figur 2D

Druckverhalten bei niedriger Permeabilität und UF>0

PB2    PB1    PD2

EP 2 640 439 B1

Figur 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20080215247 A1
- WO 2006011009 A2
- EP 1175917 A1
- US 7131956 B1